# EUROPEAN PATENT APPLICATION

(11) **EP 1 010 754 A1**
(43) Date of publication of application: **21.06.2000**
(21) Application number: 98941690.4
(22) Date of filing: 03.09.1998
(51) Int. Cl.: C12N 1/20, C09K 17/00, C05F 11/08, A01N 63/00

(54) **NOVEL MICROORGANISM**

(30) Priority: 03.09.1997 JP 23797497; 06.03.1998 JP 5488298; 06.05.1998 JP 12342698
(71) Applicant: WASHI KOSAN CO., LTD., Tokyo 108-0074 (JP)
(72) Inventor: ONO, Kotaro, Fukui 910-0024 (JP); YAMANAKA, Noriaki, Osaka 573-1103 (JP); WATANABE, Katsuyo, Tokyo 108-0074 (JP)
(74) Representative: Strehl, Peter, Dipl.-Ing.
(86) International application number: PCT/JP98/03941
(87) International publication number: WO 99/11756

(57) **Abstract**

A novel microorganism which has no induction period, immediately initiates the exponential growth, and is nonhemolytic, such as Bacillus sp. OYK-01-600 (FERM BP-6394). OYK bacteria per se have antimicrobial activities against toxic bacteria (such as Staphylococcus aureus, including methicillin-resistant Staphylococcus aureus, pathogenic Escherichia coli including O-157, Legionella pneumophila, and Klebsiella pneumoniae). A difference in growth rate can inhibit bacteria which emit an offensive odor and many noxious infection bacteria. In the utilization for the production of compost, a rapid temperature rise of a fermentation tank caused by the most important intitial heat generation facilities the production of full-ripe compost.

## Description

### TECHNICAL FIELD

The present invention relates to a novel nonhemolytic microorganism and to its utilization, and more particularly to a novel microorganism which expresses not only high antimicrobial activity against those noxious bacteria which give off offensive odors in the course of proliferation (for example, Staphylococcus aureus, Klebsiella neumoniae, etc.) and, as such, is capable of getting rid of the malodor originating from effluent treatment and facilities, but also high antimicrobial activity against other toxic bacteria (for example, meticillin-resistant Staphylococcus aureus, pathogenic Escherichia coli O-157, Legionella pneumophila, etc.), thus finding application in a broad spectrum of uses and to the utilization thereof.

It should be understood that the antimicrobial activity of the novel microorganism according to the present invention is derived not only from the extracellular and intracellular secretions of this novel microorganism but also from the following mechanism.

The novel microorganism of the present invention proliferates at an unusually high growth rate. Thus, at an incubation temperature of 20∼40°C, it multiplies from an initial population of 10³ cells/g (ml) to a population 10⁶ cells/g (ml) in about 2 hours and further to 10⁸ cells/g (ml) within 6 hours. Whereas growth of ordinary or adventitious bacteria occurs only after the so-called induction period, that is to say an acclimatization period (the time which a microorganism placed in a new environment requires for its being acclimatized to the new environment) of about 6 hours, the novel microorganism of the present invention has substantially no "induction period" as mentioned above but undergoes cell division and multiplies rapidly in an explosive manner after initiation of culture. As the microorganism of the present invention avariciously digests and assimilates available nutrients and grows explosively while said other adventitious bacteria are still in the induction period of growth, proliferation of the adventitious bacteria is inhibited and the above-mentioned antimicrobial activity against such adventitious bacteria is expressed. Thus, this novel microorganism having substantially no induction period and starting to grow immediately after commencement of culture inhibits growth of noxious bacteria.

Meanwhile, among bacteria, there are thermophilic bacteria which grow at comparatively high temperatures (for example, actinomyces belonging to the genera Micromonospora, Nocardia, Streptomyces, etc. and hyphomyces belonging to the genera Alternalia, Aspergillus, Ketomium, etc.). Thus, the microorganism of the present invention grows explosively after a brief induction period, the growth being accompanied by generation of heat which elevates the internal temperature of the culture system to about 50∼60°C, after which said thermophilic organisms begin to multiply and thereby increase the temperature of the system further to, for example, as high as close to 100°C even when the external atmospheric temperature is about 30°C.

### BACKGROUND TECHNOLOGY

Recent years have witnessed what may be called the heyday of the technology utilizing microorganisms, inclusive of the waste water treatment technology. While a large variety of microorganisms are utilized in waste water treatment, the following can be reckoned as representative organisms. Thus, as the bacteria used, Zooglea, Sphaerotilus, etc. can be mentioned; as the protozoa used, Rhizopoda, Mastigophora, Ciliata, etc. can be mentioned; and as the algae, Cyanophyceae, Chlorophyceae, Diatomeae, etc. can be mentioned.

These microorganisms are capable of decomposing organic matter and some of inorganic matter but tend to induce, in decomposing them, the production of malodors (ammonia odor, hydrogen sulfide odor) and malodor-emitting substances, and the malodors generated from waste water treatment plants have frequently been reported as a social problem. Thus, even if the decomposition of organic and inorganic substances were efficiently achieved, the malodors originating from such treatment facilities remained to be dealt with. Particularly, the active sludge formed in a sedimentation tank becomes progressively anaerobic and, as anaerobic bacteria grow, they emit a copious malodor. In the course of recycling such an active sludge to the aeration tank, a considerable spread of noxious odor drifts around the effluent treatment plant.

With the genesis of the foregoing problem by way of background, the inventors of the present invention explored earnestly for a new useful microorganism and ultimately succeeded in isolating from soil a novel useful microorganism which does not cause evolution of the malodors of hydrogen sulfide, methylmercaptan, etc. in the decomposition of organic matter and has antimicrobial activity not only against the bacteria which grow with emission of malodors, such as Staphylococcus aureus and Klebsiella pneumoniae, but also against meticillin-resistant S. aureus, pathogenic E. coli. O-157, Legionella spp., Pseudomonas aeruginosa and so forth. They found, also, that this novel microorganism can be utilized in a large variety of uses and have ultimately developed the present invention.

### DISCLOSURE OF INVENTION

The novel microorganism of the present invention is a nonhemolytic Bacillus subtilis-related microorganism which is not less than 4 times as large as the type culture strain of Bacillus subtilis (IFO3134) in cell volume and grows substantially without an induction period in an early stage of culture, for example showing an increase in population from an initial count of 10³ cells/ml to a count of not less than 10⁷ cells/ml in 4 hours when it is subjected to nutrient broth shake culture at 37°C. As the synergism of those characteristics, the metabolic energy generated by its growth reaches not less than 1×10⁴ times as compared with said type culture strain of B. subtilis. As such, this microorganism expresses antimicrobial activity against Staphylococcus aureus, Klebsiella pneumoniae, meticillin-resistant S. aureus, pathogenic E. coli O-157, Legionella spp., Pseudomonas aeruginosa, Fusarium spp., koji mold (Aspergillus oryzae), blue mold (Penicillium spp.), Trichophyton spp., and Rhyzopus spp.

As examples of the Bacillus subtilis-related microorganism, which is the novel microorganism of the present invention, the following OYK strains (deposited under the international convention with Patent Microorganism Deposit Center, National Institute of Bioscience and Human Technology, Agency of Industrial Science and Technology, the Ministry of International Trade and Industry, Japan) can be mentioned.
(1) Bacillus sp. OYK-01-600 (FERM BP-6394),
(2) Bacillus sp. OYK-03-600 (FERM BP-6395),
(3) Bacillus sp. OYK-04-000 (FERM BP-6396).

In the following description, each of OYK-01-600, OYK-03-600 and OYK-04-000 will be referred to briefly as the OYK strain.

The OYK strain digests protein and grows luxuriantly. Moreover, the OYK strain is characterized in that it does not produce hemolysin and, therefore, is highly safe to the human being. The safety of the strain has been confirmed by administering it to animals by four routes, namely intravenous, airway, oral and percutaneous.

Furthermore, because the OYK strain has antimicrobial activity against Staphylococcus aureus, Klebsiella pneumoniae, meticillin-resistant Staphylococcus aureus, Pseudomonas aeruginosa, Legionella spp., Escherichia coli O-157, Fusarium spp. (red mold), etc., it can be utilized in a variety of uses by exploiting the potentiation of its effect through administration in a high concentration. In addition, since growth of the OYK strain is not accompanied by the production of methylmercaptan, hydrogen sulfide and trimethylamine which are sources of odors particularly noxious to humans, the rewarding effect of this strain can be expressed in applications requiring deodorization.

Since generally there is the tendency that the strain of microorganism existing in a predominant population tends to grow with preponderance, the inoculum size used initially is of great importance. The preferred concentration of the inoculum for use in the present invention is 10⁶-10⁹ cells/g (ml).

The OYK strain is comparatively large in size among various strains of B. subtilis. For example, it is about 4 times (by volume) larger than the type culture strain of Bacillus subtilis [IFO 3134 (0.4∼0.6×1.5∼3.2 µm)]. Moreover, the OYK strain has a greater initial growth potential after the start of culture and thereby produces higher metabolic energies (whereas the type culture strain multiplies only 6-fold in 4 hours after the start of culture, the OYK strain grows as many as about 80,000 times). Moreover, being a facultative anaerobe, the strain is able to grow actively under both aerobic and anaerobic conditions. Therefore, it increases the temperature of organic wastes quickly in the production of a compost to promote growth of the thermophilic actinomyces and hyphomyces and thereby reduce the compost ripening time and give rise to a full-ripe compost.

Composts are used, of course, for growing farm crop plants and since the microorganisms used for compost formation are apt to be absorbed by crop plants and humans, the safety of the microorganisms used is very important. In the full-ripe compost which has experienced a high heat (70-80°C), not only the seeds of weeds present in the excreta but also infectious pathogens have been completely killed, i.e. disinfected stated differently. The compost thus obtained has been utilized as the bedding material in beef cattle barns. The full-ripe compost spread as bedding comfort cattle so that the cattle sleep well. On an uncomfortable bedding material, beef cattle keep standing without lying down and, for that reason, reportedly yield a hard grade of meat. Therefore, this application is attracting interest as a new market for full-ripe composts. In view of this aspect, it is recommended to use microorganisms with proven safety, namely OYK strains.

The novel microorganism of the present invention has excellent antimicrobial and deodorant properties. Therefore, by using it in effluent treatment facilities, the effluents can be treated without emission of malodors. Moreover, because this organism grows at an extremely high speed, composts can be produced quickly even in cold weather during the winter months and provided in the full-ripe form.

Furthermore, an antimicrobial substance can be purified from the novel microorganism of the present invention. Since the very microorganism has been isolated and screened from among soil organisms which are generally considered to be only sparingly toxic, the antimicrobial substance secreted by the particular microorganism is also considered relatively safe to humans. Therefore, the substance not only enables antibacterial processing in fields where no prior art can be applied, such as prevention of growth of adventitious microorganisms in diapers, sanitary napkins or bath water, but also enables antimicrobial processing (free from the risk for allergy) of various articles in the textile field (apparels, towels, etc.) with mild antimicrobial effects not found in the conventional bacteriostatic articles.

### [Bacterial cell preparation]

The Bacillus subtilis-related microorganism according to the present invention can be put to use either as it is or in the sporulated form but in order to insure a rapid rise-up of the growing speed (rapid increase in concentration) and provide a form easy to use, it is preferably used in the form of a cell preparation obtainable by supporting the bacteria in an enrichment matrix by entrapping, deposition or incorporation.

The enrichment matrix is preferably composed predominantly of bean protein. The protein source which can be used includes soybean, defatted soybean, processed soybean products such as soybean curd refuse, etc., soybean cake, soy-milk which is obtainable by squeezing soybeans and its concentrate or freeze-dried preparation, a dispersion or solution of milled soybean in a solvent such as water, cereal gluten, milk casein, gelatin obtainable by treating collagen in hot water, and fish protein obtainable from fish meal, among others. As other enrichment materials, rice bran, wheat bran, potato, brewer's yeast, sugar cane, etc. can also be mentioned.

When a processed soybean product, for instance, is used as the enrichment matrix, a high-solid product is directly inoculated with the microorganism and, then, milled and dried for use. A liquid preparation is first made viscous by removing water to some extent and, then, inoculated. The inoculum consists of viable cells inclusive of the culture broth.

The form of cell preparation is not particularly restricted but may for example be a powder, granules, a sheet, or a shaped article.

The additives for the enrichment material include various porous substances which provide for an aerobic environment for the novel microorganism of the invention, such as coffee grounds, sawdust, perlite, zeolite and activated charcoal, among others.

The microorganism requires for growth the four elements of carbon, nitrogen, phosphorus and sulfur in a well-balanced ratio and soybean satisfies this requirement.

Furthermore, the cell preparation according to the present invention can be supplemented with a powder of the sporulated form of a thermophilic organism (e.g. actinomycetes and hyphomycetes) which grows in a high temperature range over 50°C.

By using the enrichment matrix mentioned above, a cell preparation insuring a bacterial concentration of 1×10⁸ cells/g ∼ 1×10¹⁰ cell/g can be provided.

The above cell preparation is added to an organic waste (garbage) and animal excreta to thereby promote fermentation for compost formation. The novel microorganism of the present invention multiplies actively in the temperature range of 10∼50°C on the average to quickly create an environment favorable for the activity of microorganisms fermenting at comparatively higher temperatures.

In the high-temperature region, the OYK strain is either killed or sporulated as the temperature increases and phagocytized as nutrient sources by thermophilic bacteria and almost completely eliminated. If the upper and lower parts of the system are exchanged (reshuffled) in the neighborhood of 60°C, the unfermented substrate will be brought into contact with the bacterial cells and an aerobic milieu will be established, and the resulting temperature increase offsets the transient temperature drop caused by reshuffling, with the result that the whole system continues to ferment until the formation of a full-ripe compost.

A bacterial cell preparation with a high density of viable cells is fed to an organic waste and aerobic fermentation is promoted by reshuffling at a suitable temperature. During aerobic fermentation, some amount of ammonia is produced but the porous additive, such as coffee grounds, adsorbs the ammonia. By the metabolic energy associated with growth of the strain, the temperature increases beyond 50°C, whereby the activity of thermophiles (actinomyces and molds) are encouraged, raising the temperature further to 70°C or higher. At this high temperature, the harmful microorganisms and insects are exterminated, the decomposition of hardly decomposable organic matter interfering with the growth of crop plants is also promoted, and the seeds of weeds present in the excreta are destroyed. As a result, a wholesome full-ripe compost necessary for organic culture can be provided.

### [Deodorant]

The novel microorganism of the present invention can be used as a deodorant against the unpleasant odors (hydrogen sulfide, methylmercaptan, triethylamine) evolved in anaerobic fermentation.

As the deodorant, the microorganism can be used as it is or in the sporulated form but a cell preparation such as that described before is preferably used. However, since the objective involved is deodorization, such a preparation is preferably provided in a geometry providing a large surface area per unit weight, for example a perforated disk, beads, a flat sheet, a polygonal article, a netting, a dendritic article, and so on. The flat sheet may be perforated by punching, drilling or other means. Provision of the deodorant in a water-soluble form is preferred because it broadens the application range.

The deodorant according to the present invention can be used either as it is or packaged in a net-like container, for example in the kitchen sink, waste water conduit (inclusive of the drain port), animal quarters and animal processing facilities (e.g. the slaughter horse), the filth disposal station in a medical establishment and its floor, or a wash tank.

The beneficial properties of the novel microorganism of the present invention which can be exploited in the application thereof as a deodorant are enumerated below.
(1) The microorganism is benign to humans and capable of forming a high-density flora.
(2) Because of the continued formation of a high-density flora, the microorganism can be cultured in a porous enrichment medium (e.g. one having an odor absorbing function).
(3) The microorganism has the property to form spores and survive in hot water.
(4) The microorganism tolerates low concentrations of residual chlorine.
(5) The microorganism does not generate a malodor in growing or, if it does, generates only a small amount of odor.
(6) When the microorganism is dispersed in an enrichment source-containing water-soluble solid matter in a highly humid environment, it exhibits a growing potential surpassing the growth of other microorganisms (adventitious bacteria) which produce malodors as they grow.
(7) The microorganism elaborates and secretes an antimicrobial substance to inhibit proliferation of adventitious bacteria present at low concentrations.
(8) If ingested, the microorganism is not hazardous to health.

### [Inhibition of growth of adventitious microorganisms in bath water]

Using the novel microorganism of the present invention, the water in the bathtub and the wall and cover of the bathtub can be protected from growth of adventitious microorganisms and deodorized.

### [Sanitary products]

The novel microorganism of the present invention can be put to use as incorporated in sanitary products. The sanitary products in the context of the present invention mean the articles for dealing with the secretions and excretions associated with physiological events in man and other animals, thus including sanitary napkins, diapers, sheets for prevention of bed sore, and underwear, among others. Among the above-mentioned sanitary products, the particularly suitable product is a sanitary napkin. Thus, since menstrual blood is absorbed by an adsorbent made of a highly hygroscopic polymer material or a fibrous material, inclusion of the microorganism of the present invention, for example an OYK strain or a cell preparation thereof, results in inhibition of growth of adventitious microorganisms inclusive of the toxic bacteria present in the menstrual blood-absorbed area and, at the same time, in deodorization of the napkin. When the novel microorganism is applied to a diaper for preventing leakage of excreta by absorption, a similar result is obtained. Thus, the novel microorganism as applied to a diaper not only inhibits proliferation of the odor-causing adventitious microorganisms present in the excreta and deodorizes the diaper but also can be expected to prevent infection by virtue of the antimicrobial activity of the novel microorganism.

A variety of methods can be contemplated for retaining an OYK strain or a powder of the OYK strain on the sanitary products mentioned above. When the bacteria can hardly be immobilized on the substrate, a powder of the bacteria may be mixed with peptone-containing physiological saline and the mixture applied with a sprayer. When the substrate is wet, the bacterial powder can be directly dusted thereon. The following alternative methods may also be employed. Thus, the bacteria may be filled into a pouch made of water-soluble sheeting and the filled pouch be attached to the sanitary product or alternatively the bacteria maybe fixed on the surface of such a water-soluble sheet with the aid of a water-soluble binder and the resulting sheet attached to the sanitary product. The water-soluble sheet material which can be used includes but is not limited to starch, casein, polyvinyl alcohol (PVA) and carboxymethylcellulose (CMC).

When a person or an animal lies on the bed for a long time, the skin in contact with the bed surface develops the so-called "bed sore". In fish, keeping them in a water tank for a long time may cause injury to the scales due to mutual contact of the fish or development of a slime on the surface of the fish body. In avian species, the surface gloss of the feathers may be lost. In small pet animals such as dogs, cats, etc. or in domestic animals such as cattle, swine, sheep and horses, the hair coat will lose a groomed appearance. These are invariably caused by an accumulation of secretions from the body surface and hairs of animals or putrefaction of food residues adhering to the body surface of fish. Thus, as secretions from the animal's body surface and hair coat accumulate and adventitious microorganisms feed on the secretions and grow, the above-mentioned abnormal events are induced. In some cases, bacteria affecting the skin, such as pseudomonal species, may grow so that the normal flora is disturbed.

The OYK strain decomposes said secretions and shows antimicrobial activity against harmful microorganisms so that, in the cases mentioned above, marked improvements can be obtained by dusting or coating the OYK microorganism on the body surface. For direct dusting or coating of the OYK strain on the skin, feather or hair coat of man or animals, it is expedient to use a solution containing the OYK microorganism or a powder of the sporulated form of the microorganism. When a powder is used, it can be directly applied using a pneumatic duster. The spores of the OYK strain applied by dusting obtain moisture from the living body, germinate and continue to multiply by digesting the secretions. The speed of proliferation is rapid without production of ammonia so that the odor is eliminated after a while and the dusted parts become supple, giving a feeling as if washed. The same effect can be obtained on the bird's feather. By adding a powder of the OYK microorganism to an aquarium in which fish is kept, the slimy coat on the body surface can be eliminated and the cure of the scale wound is rapid. This treatment is particularly suitable for fresh-water fish. The concentration of the OYK microorganism to be applied by dusting or coating may be 1×10⁶∼1×10⁸ cells/g and the microorganism can be used in combination with an enrichment material for the microorganism.

### [Antimicrobial goods]

The antimicrobial goods or articles according to the present invention include, in addition to the above-mentioned sanitary products, casings for OA equipment and devices, stationery goods such as desk pads and pencil cases, and communications equipment such as FAX and telephone sets.

### [Warming equipment or devices]

The warming equipment includes plane heaters, among others. It has been confirmed that the bacterial cell preparation increases its temperature to about 40∼50°C when exposed to a humidity of approximately 50∼60%. Therefore, when the bacterial cell preparation of the present invention is packaged in a sealed container or bag, for instance, and, then, the package is unsealed, the bacteria exposed to moisture begins to multiply so that the metabolic energy associated with this growth can be utilized as an energy source for said plane heater.

### BRIEF DESCRIPTION OF THE DRAWINGS

Fig. 1 is a schematic diagram showing the effluent treatment equipment and its capacity in an effluent disposal plant used in a deodorization test.
Fig. 2 is a diagrammatic representation showing the relative growth speeds of the OYK microorganism and the type culture strain of B. subtilis.
Fig. 3 is a diagrammatic view showing the time course of temperature in compost formation.
Fig. 4 is another diagrammatic view showing the time course of temperature in compost formation.
Fig. 5 is a diagrammatic view showing the time course of temperature in outdoor compost formation.
Fig. 6 is a diagrammatic view showing the time course of temperature in compost formation in a field test.
Fig. 7 is a perspective view showing the geometry of the deodorant according to the invention.
Fig. 8 is a sectional view showing the molding of a solid deodorant according to the invention.
Fig. 9 is a sectional view showing an agar medium as immobilized.
Fig. 10 is a sectional view of the OYK strain-carrying support.
Fig. 11 is a sectional view of the OYK strain-carrying support to which a float means has been attached.

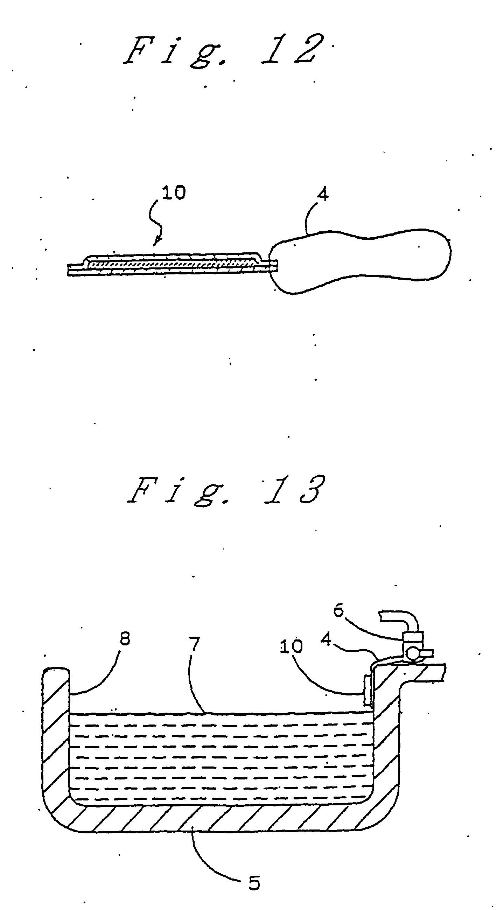

Fig. 12 is a sectional view of the OYK strain-carrying support according to another embodiment.
Fig. 13 is a sectional view showing an example of application of the OYK strain-carrying carrier to a bathtub.
Fig. 14 is a sectional elevation view of an equipment for utilizing the energy of metabolism as a thermal energy source.

### BEST MODE FOR CARRYING OUT THE INVENTION

The present invention is now described by way of examples. It should be understood that these examples are merely illustrative and by no means limitative of the scope of the invention.

### A. Screening for novel microorganisms

Three strains of microorganisms having antimicrobial activity against bacteria whose growth is accompanied by emanation of malodors were isolated from the soil. Soil microorganisms are generally considered to be only sparingly toxic and the above 3 OYK strains isolated from the soil may also be considered safe (benign) to the human body.

### B. Identification of the strains

The microbiological characteristics of the strains isolated and screened by the present inventors were differentiated according to the following literature (1) and (2).
(1) Bergey's Mannual of Determinative Bacteriology, Vol. 2 (1986), Williams & Wilkins, U.S.A.
(2) R. E. Gordon, W. C. Haynes, C. H. Pang. (1973). The Genus Bacillus. Agr. Handbook No. 427, United States Department of Agriculture, Washington D.C.

Tests were performed by the under-mentioned procedures.

### 1. Morphologic characters (Results are shown below in Table 1)

### (1) Cell size

### (2) Cell form

### (3) Polymorphism

The observation is made using an electron microscope at ×5,000∼30,000 magnification. Each test strain is cultured on an agar plate at 37°C × 1 day and the colony formed is harvested. The broth agar medium used is composed of meat extract 5 g, peptone 10 g, NaCl 5 g, agar 15 g and distilled water 1,000 ml.

### (4) Motility

1) Flagella are strained by the method of Nishizawa and Sugawara and observed with an electron microscope (×10,000). The first solution is composed of tannic acid 100 ml, ferric chloride solution JP 1.5 ml, formalin 2 ml, 1% NaOH 1 ml, and distilled water 100 ml, and the second solution is composed of silver nitrate 2 g, aqueous ammonia JP q.s., and distilled water 100 ml.
2) Stab culture is performed using a semi-fluid broth agar medium for stab culture and the evaluation is made according to the turbidity of the entire medium. The semi-fluid both agar medium is composed of meat extract 5 g, peptone 10 g, NaCl 5 g, agar 5 g and distilled water 1,000 ml.

### (5) Sporulation

1) The culture obtained by shake culture on broth agar is heated at 85°C × 15 minutes and transferred to a broth agar plate by the spread-plating dilution technique and the evaluation is made according to the formation of colonies. The broth agar medium used is composed of meat extract 3 g, peptone 5 g and distilled water 1,000 ml.
2) Spores are stained by the method of Wirtz (modified Schaeffer-Fulton method) and the evaluation is made according to red staining for cells and green staining for spores. The red color of cells is developed with 0.5% aqueous safranine and the green color of spores is developed with 5% aqueous malachite green.

### (6) Sporangium form

### (7) Spore form

### (8) Position of spore formation

### (9) Spore size

Spores are stained by the method of Writz (modified Schaeffer-Fulton method) and observed with an electron microscope at ×5,000 magnification. The test strain is cultured on a broth agar plate at 37°C × 1 day and harvested from the cold colonies stored in a refrigerator at 4°C for 1 day.

### (10) Gram-stain

The modified Hucker method is used. The positive test is a violet stain with crystal violet and the negative test is a red stain by counter staining with safranin red. The test strain is cultured on a broth agar plate at 37°C × 1 day and harvested from the colony formed.

### (11) Acid-fastness

The Ziiehl-Neeisen staining method is used. The positive test is a red stain with fuchsine-phenol and the negative test is a green stain by counter staining with malachite green. The test strain is cultured on a broth agar plate at 37°C × 1 day and harvested from the colony formed.

**Table 1**

| Morphological characters | | | |
|---|---|---|---|
| Morphological observation parameters | Bacillus sp. OYK-01-600 (FERM BP-6394) | Bacillus sp. OYK-03-600 (FERM BP-6395) | Bacillus sp. OYK-04-000 (FERM BP-6396) |
| (1) Cell size | 0.7-0.9 × 2.5-5.1 µm | 0.7-0.9 × 2.5-5.1 µm | 0.7-0.9 × 2.5-5.1 µm |
| (2) Cell form | Rod | Rod | Rod |
| (3) cell polymorphism | None | None | None |
| (4) Motility | Motile | Motile | Motile |
| (5) Sporulation | Spore-forming | Spore-forming | Spore-forming |
| (6) Sporangium form | Non-distending | Non-distending | Non-distending |
| (7) Spore form | oblong | oblong | oblong |
| (8) Position of spore formation | Central -subterminal | Central -subterminal | Central -subterminal |
| (9) Spore size | 0.8-1.0 × 1.5-1.8 µm | 0.8-1.0 × 1.5-1.8 µm | 0.8-1.0 × 1.5-1.8 µm |
| (10) Gram-stain | Positive | Positive | Positive |
| (11) Acid-fastness | Positive | Positive | Positive |

### 2. Cultural characters on various media (Results are shown below in Tables 2 and 3)

### (1) Broth agar plate culture

### [Medium composition] (in 1,000 ml of distilled water)

Meat extract 5g, sodium chloride 5 g, peptone 10 g, agar 15 g (pH 7.0±0.1)

### [Cultural conditions]

The medium is set in sterilized dishes and the test strain is streak-cultured. 37°C × 3 days.

### (2) Broth agar slant culture

### [Medium composition] (in 1,000 ml of distilled water)

The same as above

### [Cultural conditions]

The medium is set in inclined position in sterilized test tubes and the test strain is streak-cultured on the medium. 37°C × 3 days.

### (3) Liquid broth culture

### [Medium composition] (in 1,000 ml of distilled water)

Meat extract 5 g, sodium chloride 5 g, peptone 10 g (pH 7.0±0.1)

### [Cultural conditions]

The medium is dispensed into sterilized test tubes and the test strain is suspended in the medium. 37°C × 3 days, under shaking at 170 cycles/min.

### (4) Gelatin stab culture

### [Medium composition] (in 1,000 ml of distilled water)

Meat extract 5 g, sodium chloride 5 g, peptone 10 g, gelatin 15 g (pH 7.0±0.1)

### [Cultural conditions]

The medium for stab culture is set in sterilized test tubes and the test strain is stab-cultured. 25°C × 7 days.

### [Observation parameters other than growth]

Liquefaction of gelatin by proteolysis.

### (5) Litmus milk liquid culture

### [Medium composition] (in 1,000 ml of distilled water)

Skim milk power 110 g, litmus solution q.s. (pH 7.0±0.1)

### [Cultural conditions]

The medium is dispensed into sterilized test tubes and the test strain is suspended in the medium. 25°C × 14 days. Stationary culture.

### [Observation parameters other than growth]

Coagulation of cow's milk due to the acid produced by decomposition of lactose
Coagulation of cow's milk due to the rennin produced by decomposition of lactose
Precipitation of cow's milk serum due to the rennin produced by decomposition of lactose

**Table 2**

| Cultural characters on various media | | | |
|---|---|---|---|
| Cultural characters examined | Bacillus sp. OYK-01-600 (FERM BP-6394) | Bacillus sp. OYK-03-600 (FERM BP-6395) | Bacillus sp. OYK-04-000 (FERM BP-6396) |
| (1) Broth agar plate culture, 37°C×3 days | The 3 mutant strains showed more or less similar cultural characters. | | |
| Growth | Very rapid, growing to attain a diameter of 2-3 mm in 1 day and 4-6 mm in 2 days. | | |
| Surface | Translucent, with slightly granulose irregularities | | |
| Color | Cream-like milk white | | |
| Size | 6-8 mm in diameter | | |
| Gloss | Slightly glistening | | |
| Form | Circular | | |
| Elevation | Flat but the mycelial pad is heavily thick | | |
| Margin | Not an entire but a fimbriated or sinuated margin | | |
| Pigment production | None | | |

**Table 3**

| Cultural characters examined | Bacilius sp. OYK-01-600 (FERM BP-6394) | Bacillus sp. OYK-03-600 (FERM BP-6395) | Bacillus sp. OYK-04-000 (FERM BP-6396) |
|---|---|---|---|
| (2) Broth agar slant culture, 37°C×3 days | The 3 mutant strains showed more or less similar cultural characters. | | |
| Growth | Luxuriant, spreading well on the slant | | |
| Surface | Translucent, with slightly granulose irregularities | | |
| Color | cream-like milk white | | |
| Gloss | Slightly glistening | | |
| Form | Flat but the mycelial pad is heavily thick | | |

| (3) Liquid broth culture, 37°C×3 days | | | |
|---|---|---|---|
| Turbidity | High | High | High |
| Surface growth | Profuse and punctate | Profuse and punctate | Profuse and punctate |
| Sediment | Moderate | Profuse | Profuse |
| (4) Gelatin stab culture, 25°C×7 days | The 3 mutant strains showed more or less similar cultural characters. | | |
| Growth | Luxuriant | | |
| Liquefaction | A 1.5 cm-deep white opaque liquefied layer from surface of medium | | |

| (5) litmus milk liquid culture, 25°C×14 days | | | |
|---|---|---|---|
| Production of acid | None | None | None |
| Coagulation | Positive | Positive | Positive |
| Separation of milk serum | Positive | Positive | Positive |

### 3. Physiological characters (1) (Results are shown below in Table 4)

### (1) Reduction of nitrate

### [Medium composition] (in 1,000 ml of distilled water)

Potassium nitrate 1 g, peptone 5 g

### [Cultural conditions]

The medium is dispensed into sterilized test tubes and a loopful of the test organism is suspended in the medium. 37°C × 5 days, under shaking at 170 cycles/min.

### [Observation parameter]

Detection of nitrite: 1 ml of α-naphthylamine solution and 1 ml of sulfanillic acid solution are thoroughly mixed with the medium. Positive test: a pink-red color.

### (2) Denitrification reaction

### [Medium composition] (in 1,000 ml of distilled water)

1) Sodium nitrate 10 g, meat extract 5 g
2) Meat extract 5 g

### [Cultural conditions]

The above media 1 and 2 are respectively dispensed into 2 sterilized test tubes and a loopful of the test organism is suspended in the medium. In one of the two tubes, liquid paraffin is overlayed in a thickness of 1∼2 cm. 37°C × 13 days. Stationary culture.

### [Observation parameter]

Anaerobic growth in the presence of nitrate: the evaluation is made according to the degree of turbidity and gas production in 4 tubes with or without nitrate and liquid paraffin.

### (3) VP test

### [Medium composition] (in 1,000 ml of distilled water)

Peptone 5 g, potassium dihydrogenphosphate 5 g, glucose 5 g

### [Cultural conditions]

The medium is dispensed into sterilized test tubes and a loopful of the test organism is suspended in the medium. 37°C × 3 days, under shaking at 170 cycles/min.

### [Observation parameter]

Acetylmethylcarbinol as a decomposition product of glucose: 0.6 ml of α-naphthol solution and 0.2 ml of 40% aqueous KOH solution are added to 1 ml of the culture. Positive test: a deep red color.

### (4) MR test

### [Medium composition] (in 1,000 ml of distilled water)

Peptone 5 g, potassium dihydrogenphosphate 5 g, glucose 5 g

### [Cultural conditions]

The medium is dispensed into sterilized test tubes and a loopful of the test organism is suspended in the medium. 37°C × 3 days, under shaking at 170 cycles/min.

### [Observation parameter]

Production of acid due to decomposition of glucose: methyl red is dripped into 1 ml of the culture. Positive test: a red color. Negative test: a yellow color.

### (5) Production of indole

### [Medium composition] (in 1,000 ml of distilled water)

Peptone 10 g, sodium chloride 5 g

### [Cultural conditions]

The medium is dispensed into sterilized test tubes and a loopful of the test organism is suspended in the medium. 37°C × 3 days, under shaking at 170 cycles/min.

### [Observation parameter]

Detection of indole: the ability to produce indole from the amino acid tryptophan. To the culture is added 1/5∼1/10 of Kovac's reagent (shown below), and the tube is swirled vigorously and allowed to stand. The upper layer of the bilayers formed is observed. Positive test: a crimson color. Negative test: a yellow color.

### 〈Kovac's Reagent〉

p-Dimthylaminobenzaldehyde 5 g, amyl alcohol 75 ml, concentrated hydrochloric acid 25 ml.

### (6) Production of hydrogen sulfide

### [Medium composition] (commercial TSI agar medium, in 1,000 ml of distilled water)

Meat extract 5 g, glucose 1 g, sodium chloride 5 g, ferric citrate 0.2 g, peptone 15 g, sodium thiosulfate 0.2 g, lactose 10 g, Phenol Red 0.002 g, sucrose 10 g, agar 15 g

### [Cultural conditions]

The medium is set in a semi-slant position within sterilized test tubes and inoculated by stabbing into the substance of the medium and smearing on the slant surface. 37°C × 1 day.

### [Observation parameter]

Detection of hydrogen sulfide: positive test: a black substance in a low position on the slant.

### (7) Hydrolysis of starch

### [Medium composition] (in 1,000 ml of distilled water)

Starch 2 g, meat extract 5 g, peptone 10 g, sodium chloride 5 g, agar 15 g

### [Cultural conditions]

The medium is set on sterilized dishes and streak culture is carried out. Room temperature × 5 days.

### [Observation parameter]

Detection of starch: an iodine-potassium iodide solution (shown below) is dripped on the dish showing growth of the test organism. Positive test: disappearance of the deep purple color.

### 〈Iodine-potassium iodide solution〉

Potassium iodide 5 g, iodine 4 g/200 ml

### (8) Liquefaction of casein

### [Medium composition] (in 1,000 ml of distilled water)

Skim milk 2 g, agar 5 g

### [Cultural conditions]

The above medium components are independently sterilized, mixed and the mixture is set on sterilized dishes. Then, streak culture is performed. 37°C × 1 day.

### [Observation parameter]

Casein residues: Positive test: a transparent zone about the streak.

### (9) Utilization of citric acid

### [Medium composition] (commercial CIT agar medium, in 1,000 ml of distilled water)

Dipotassium hydrogenphosphate 1 g, monoammonium hydrogeaphosphate 1 g, sodium citrate 2 g, magnesium sulfate 0.2 g, sodium chloride 5 g, BTB 0.024 g, agar 15 g

### [Cultural conditions]

The medium is set on sterilized dishes and streak culture is performed. 37°C × 1∼3 days.

### [Observation parameter]

Growth by utilizing citric acid as a sole carbon source: Positive test: blue discoloration of the medium or growth of the organism.

### (10) Pigment production

### [Medium composition] (in 1,000 ml of distilled water)

Peptone 20 g, glycerin 10 g, K₂SO₄ 10 g, MgCl₂ 1.4 g, agar 15 g

### [Cultural conditions]

The medium is set on sterilized dishes and streak culture is performed. 25°C × 5 days.

### [Observation parameter]

Production of pigments: Selective coloration of the medium around colonies indicates production of insoluble pigments, while coloration of the whole medium indicates production of soluble pigments.

### (11) Urease

### [Medium composition] (in 1,000 ml of distilled water)

Urea 20 g, peptone 2 g, glucose 1 g, sodium chloride 5 g, potassium dihydrogenphosphate 2 g, 0.2% Phenol Red 6 ml, agar 15 g (Note: agar is autoclaved at 121°C × 15 min. and the other components are sterilized using a bacterial filter)

### [Cultural conditions]

The medium is set in an inclined position within sterilized test tubes and smear culture is performed. 37°C × 1 day.

### [Observation parameter]

Detection of ammonia: Positive test: the medium turns red.

### (12) Oxidase

### [Medium composition] (in 1,000 ml of distilled water)

Meat extract 5 g, peptone 10 g, sodium chloride 5 g, agar 15 g

### [Cultural conditions]

The medium is set on sterilized dishes and streak culture is performed. 37°C × 1 day.

### [Observation parameter]

Presence of cytochrome: A 1% aqueous solution of dimethyl-p-phenylenediamine is dripped on colonies on the plate. Positive test: the drip color turns pink and, then, black.

### (13) Catalase

### [Medium composition] (in 1,000 ml of distilled water)

Meat extract 5 g, peptone 10 g, sodium chloride 5 g, agar 15 g

### [Cultural conditions]

The medium is set on sterilized dishes and streak culture is performed. 37°C × 1 day.

### [Observation parameter]

Presence of catalase: The presence of the enzyme which catalyzes the decomposition of hydrogen peroxide. One drop of 3% H₂O₂ solution is placed on a glass slide and a loopful of the test organism is thoroughly mixed therein. Positive test: copious or sustained formation of oxygen bubbles.

**Table 4**

| Physiological characters (1) | | | |
|---|---|---|---|
| | Bacillus sp. OYK-01-600 (FERM BP-6394) | Bacillus sp. OYK-03-600 (FERM BP-6395) | Bacillus sp. OYK-04-000 (FERM BP-6396) |
| (1) Reduction of nitrate | Positive | Positive | Positive |
| (2) Denitrification reaction | Negative | Negative | Negative |
| (3) VP test | Positive | Positive | Positive |
| (4) MR test | Negative | Negative | Negative |
| (5) Production of indole | Negative | Negative | Negative |
| (6) Production of hydrogen sulfide | Negative | Negative | Negative |
| (7) Hydrolysis of starch | Positive | Positive | Positive |
| (8) Liquefaction of casein | Positive | Positive | Positive |
| (9) Utilization of citric acid | Positive | Positive | Positive |
| (10) Production of pigments | Negative | Negative | Negative |
| (11) Urease | Negative | Negative | Negative |
| (12) Oxidase | Positive | Positive | Positive |
| (13) Catalase | Positive | Positive | Positive |

### 4. Physiological characters (2) (Results are shown below in Table 5 and Table 6)

### (1) pH-dependent growth

### [Medium composition] (commercial nutrient broth, in 1,000 ml of distilled water)

Meat extract 3 g, peptone 5 g, pH control agent (acid: H₂SO₄ 1 ml/100 ml; alkali: NaOH 4 g/1,000 ml). The pH is varied in 15 steps between 3.6 and 10.9.

### [Cultural conditions]

The medium is dispensed into sterile test tubes and a loopful of the test organism is suspended in the medium. 37°C × 1 day, under shaking at 170 cycles/min.

### [Observation parameter]

### Growth

- ++: Good growth
- +: Moderate growth
- -+: Slight growth
- -: Scanty growth
- --: No growth at all

### (2) Temperature-dependent growth

### [Medium composition] (commercial broth agar medium, in 1,000 ml of distilled water)

Meat extract 5 g, sodium chloride 5 g, peptone 10 g, agar 15 g (pH 7.0±0.1)

### [Cultural conditions]

The medium is set on sterilized dishes and streak culture is performed.

The temperature is varied serially, 4, 10, 20, 30, 37, 40 and 50°C, each × 1 day.

### [Observation parameter]

### Growth

- ++: Good growth
- +: Moderate growth
- -+: Slight growth
- -: Scanty growth
- --: No growth at all

### (3) Attitude toward oxygen

### [Medium composition] (commercial broth agar medium, in 1,000 ml of distilled water)

Meat extract 5 g, sodium chloride 5 g, peptone 10 g, agar 15 g (pH 7.0±0.1)

### [Cultural conditions]

The test organism and the medium are mixed and set in a high layer in sterilized test tubes and incubated. 37°C × 1 day.

### [Observation parameter]

### Growth under aerobic/anaerobic conditions

- Growth exclusively on surface: aerobic
- Growth on surface and in substance: facultatively anaerobic
- Growth exclusively in substance: oligatively anaerobic

### (4) O-F test

### [Medium composition] (in 1,000 ml of distilled water)

Peptone 2 g, sodium chloride 5 g, K₂HPO₄ 0.3 g, agar 3 g, 0.2% BTB 15 ml, glucose 10 g (Note: glucose is sterilized with a bacterial filter and the other components are sterilized by autoclaving at 121°C × 15 min.)

### [Cultural conditions]

The medium is distributed and set in a high layer in 2 sterilized test tubes. The test organism is stab-cultured in each tube. The medium in one tube is overlayed with liquid paraffin in a thickness of 1-2 cm. 37°C × 3∼4 days.

### [Observation parameter]

### Oxidative or fermentative glycolysis

- "O": oxidative glycolysis (yellowing under anaerobic conditions only)
- "F": fermentative glycolysis (yellowing under both aerobic and anaerobic conditions)

### (5) PPA test

### [Medium composition] (in 1,000 ml of distilled water)

Yeast extract 3 g, phenylalanine 2 g, sodium phosphate 1 g, sodium chloride 5 g, agar 15ml

### [Cultural conditions]

The medium is set in an inclined position in sterilized test tubes and smear culture is performed. 37°C × 1 day.

### [Observation parameter]

Deamination of phenylalanine to phenylpyruvic acid: a 10% aqueous solution of ferric chloride is dripped. Positive test: change of color to green.

### (6) Utilization of propionic acid

### [Medium composition] (in 1,000 ml of distilled water)

Magnesium sulfate 0.2 g, sodium propionate 2 g, dipotassium hydrogenphosphate 1 g, monoammonium phosphate 1 g, sodium chloride 5 g, agar 10 g, 0.2% BTB solution 12 ml

### [Cultural conditions]

The medium is set in sterilized dishes and streak culture is performed. 37°C × 1 day.

### [Observation parameter]

Growth by utilizing propionic acid as a sole carbon source: Positive test: change of medium color to blue or growth of the organism

### (7) Decomposition of tyrosine

### [Medium composition] (in 1,000 ml of distilled water)

L-tyrosine 5 g, meat extract 3 g, peptone 5 g, agar 15 g (Note: tyrosine and nutrient agar are separately steam-sterilized and mixed)

### [Cultural conditions]

The medium is set in sterilized dishes and streak culture is performed. 37°C × 7∼14 days.

### [Observation parameter]

Decomposition of tyrosine: Positive test: dissolution of crystalline tyrosine beneath the colony.

### (8) Egg yolk reaction

### [Medium composition] (in 1,000 ml of distilled water)

Peptone 10 g, Na₂HPO₄ 5 g, KH₂PO₄ 1 g, NaCl 12 g, MgSO₄ 0.1 g, glucose 2 g, egg yolk 15 ml (Note: the components other than egg yolk are steam-sterilized, the egg yolk is aseptically aspirated and added, and the mixture is ripened in the refrigerator for 24 hours. The broth not containing egg yolk is also provided)

### [Cultural conditions]

The above media with and without egg yolk are dispensed into 2 sterilized test tubes and a loopful of the test organism is suspended in each medium. 37°C × 7 days. Observation is made on days 1, 3, 5 and 7. Shake culture at 170 cycles/min.

### [Observation parameter]

Occurrence of white precipitates: Positive test: white precipitates in the lower part of the tube and on the surface of the medium in the presence of egg yolk in contrast to the absence of egg yolk.

### (9) Growth in the presence of 2% NaCl

### [Medium composition] (in 1,000 ml of distilled water)

Meat extract 3 g, peptone 5 g, NaCl 120 g

### [Cultural conditions]

The medium is dispensed into sterilized test tubes and a loopful of the test organism is suspended in the medium. 37°C × 14 days, under shaking at 170 cycles/min.

### [Observation parameter]

### Growth

- ++: Good growth
- +: Moderate growth
- -+: Slight growth
- -: Scanty growth
- --: No growth at all

### (10) Growth in the presence of 5% NaCl

### (11) Growth in the presence of 7% NaCl

### (12) Growth in the presence of 12% NaCl

### (13) Growth in the presence of 20% NaCl

### [Medium composition] (in 1,000 ml of distilled water)

The amount of NaCl in the above medium composition for (9) is altered to 70 g, 120 g and 200 g.

### [Cultural conditions]

The same as for (9).

### [Observation parameter]

The same as for (9).

### (14) Growth in the presence of lysozyme

### [Medium composition] (in 1,000 ml of distilled water)

Meat extract 3 g, peptone 5 g, lysozyme 0.1 g (lysozyme is boiled in 0.01-N HCl for 20 min. and added to nutrient broth)

### [Cultural conditions]

The medium is dispensed into sterilized test tubes and a loopful of the test organism is suspended in the medium. 37°C × 14 days, under shaking at 170 cycles/min.

### [Observation parameter]

### Growth:

- ++: Good growth
- +: Moderate growth
- -+: Slight growth
- -: Scanty growth
- --: No growth at all

### (15) Production of hemolysin

### [Medium composition] (commercial sheep blood agar medium, in 1,000 ml of distilled water)

Pancreatic digest of casein 14.5 g, papaic digest of soybean meal 5.0 g, sodium chloride 5.0 g, growth factors 1.5 g, agar 14.0 g, sheep blood, defibrinated 5.0%

### [Cultural conditions]

The medium is set in sterilized dishes and streaked with the test organism. 37°C × 1 day.

### [Observation parameter]

### Production of hemolysin

- α-hemolysis: a green zone around the colony (denaturation of hemoglobin)
- β-hemolysis: a transparent zone around the colony (disruption of red blood cell membrane)

**Table 5**

| Physiological characters (2) | | | |
|---|---|---|---|
| | Bacillus sp. OYK-01-600 (FERM BP-6394) | Bacillus sp. OYK-03-600 (FERM BP-6395) | Bacillus sp. OYK-04-000 (FERM BP-6396) |
| (1) pH-dependent growth | | | |
| pH 3.6 | -- | -- | -- |
| pH 4.1 | -- | -- | -- |
| pH 4.9 | -- | -- | -- |
| pH 5.4 | - | - | - |
| pH 6.1 | ++ | ++ | ++ |
| pH 6.5 | ++ | ++ | ++ |
| pH 7.1 | ++ | ++ | ++ |
| pH 7.5 | ++ | ++ | ++ |
| pH 8.0 | ++ | ++ | ++ |
| pH 8.6 | ++ | ++ | ++ |
| pH 9.1 | + | ++ | ++ |
| pH 9.5 | + | + | + |
| pH 10.2 | + | + | + |
| pH 10.9 | -+ | -+ | -+ |

| (2) Temperature-dependent growth | | | |
|---|---|---|---|
| 4°C | -- | -- | -- |
| 10°C | -+ | -+ | -+ |
| 20°C | + | + | + |
| 30°C | ++ | ++ | ++ |
| 37°C | ++ | ++ | ++ |
| 40°C | ++ | ++ | ++ |
| 50°C | + | + | + |

**Table 6**

| | Bacillus sp. OYK-01-600 (FERM BP-6394) | Bacillus sp. OYK-03-600 (FERM BP-6395) | Bacillus sp. OYK-04-000 (FERM BP-6396) |
|---|---|---|---|
| (3) Attitude toward oxygen | Facultatively anaerobic | Facultatively anaerobic | Facultatively anaerobic |
| (4) O-F test | F | F | F |
| (5) PPA test | Negative | Negative | Negative |
| (6) Utilization of propionic acid | Negative | Negative | Negative |
| (7) Decomposition of tyrosine | Negative | Negative | Negative |
| (8) Egg yolk reaction | Negative | Negative | Negative |
| (9) Growth in the presence of 2% NaCl | ++ | ++ | ++ |
| (10) Growth in the presence of 5% NaCl | + | - | + |
| (11) Growth in the presence of 7% NaCl | - | -- | - |
| (12) Growth in the presence of 12% NaCl | -- | -- | -- |
| (13) Growth in the presence of 20% NaCl | -- | -- | -- |
| (14) Growth in the presence of lysozyme | ++ | ++ | ++ |
| (15) Production of hemolysin | Negative | Negative | Negative |

### 5. Production of acid and gas from carbon sources (Results are shown below in Table 7)

### (1) D-glucose

### [Medium composition] (in 1,000 ml of distilled water)

Peptone 1 g, sodium chloride 5 g, agar 10 g, 0.2% BTB 15 ml, D-glucose 8 g

### [Cultural conditions]

The medium is dispensed into sterilized test tubes and set in a high layer. Then, stab culture is performed. 37°C × 14 day.

### [Observation parameter]

### Production of acid: evaluated according to the degree of yellowing of BTB reagent

- ++: Copious production
- +: Moderate production
- -+: Mild production
- -: No production

### Production of gas: evaluated according to cracks in the high layer

- ++: Copious production
- +: Moderate production
- -+: Mild production
- -: No production

### (2) L-arabinose

### (3) D-xylose

### (4) D-mannit

### (5) D-mannose

### (6) D-galactose

### (7) D-sorbitose

### (8) Inositol

### (9) Trehalose

### (10) Lactose

### (11) Maltose

### (12) Fructose

### [Medium composition] (in 1,000 ml of distilled water)

The carbohydrate (D-glucose) in the above-mentioned recipe for (1) is replaced with the carbohydrates (2)∼(12).

### [Cultural conditions]

The same as for (1).

### [Observation parameters]

The same as for (1).

**Table 7**

| Production of acid and gas from carbon sources | | | | | | |
|---|---|---|---|---|---|---|
| | Bacillus sp. OYK-01-600 (FERM BP 6394) | | Bacillus sp. OYK-03-600 (FERM BP 6395) | | Bacillus sp. OYK-04-000 (FERM BP-6396) | |
| | Acid | Gas | Acid | Gas | Acid | Gas |
| (1) D-glucose | ++ | - | ++ | - | ++ | - |
| (2) L-arabinose | ++ | - | ++ | - | ++ | - |
| (3) D-xylose | + | - | + | - | + | - |
| (4) D-mannit | + | - | + | - | + | - |
| (5) D-mannose | -+ | - | -+ | - | -+ | - |
| (6) D-galactose | - | - | - | - | - | - |
| (7) D-sorbitose | - | - | - | - | - | - |
| (8) Inositol | + | - | + | - | + | - |
| (9) Trehalose | + | - | + | - | + | - |
| (10) Lactose | -+ | - | -+ | - | -+ | - |
| (11) Maltose | - | - | - | - | - | - |

### 6. Identification Results

The three strains OYK-01-600, OYK-03-600 and OYK-04-000 were classified according to the following literature.
(1) Bergey's Mannual of Determinative Bacteriology, Vol. 2 (1986), Williams & Wilkins U.S.A.
(2) R. E. Gordon, W. C. Haynes, C. H. Pang. (1973), The Genus Bacillus. Agr. Handbook No. 427, United States Department of Agriculture, Washington D.C.

**Table 8**

| Identification results (1)-Generic differentiation | | | | |
|---|---|---|---|---|
| | Bacillus | OYK-01-600 | OYK-03-600 | OYK-04-000 |
| (1) Rods | + | + | + | + |
| (2) Diameter ≧ 2.5 µm | - | - | - | - |
| (3) Filaments | - | - | - | - |
| (4) Curvature of rods or filaments | - | - | - | - |
| (5) Cocci in tetrads or packets | - | - | - | - |
| (6) Endospore formation | + | + | + | + |
| (7) Motility | + | + | + | + |
| (8) Positive Gram-stain in growth phase | + | + | + | + |
| (9) Obligatory aerobic | D | - | - | - |
| (10) Facultatively anaerobic or microaerophilic | D | + | + | + |
| (11) Anaerobic | - | - | - | - |
| (12) Fermentation of homo-lactic acid | D | + | + | + |
| (13) Reduction of sulfate | - | - | - | - |
| (14) Catalase | + | + | + | + |
| (15) Oxidase | D | + | + | + |
| (16) Production of acid from glucose | + | + | + | + |
| (17) Reduction of nitrate | D | + | + | + |
| (18) Mol % G+C | 32-69 | ND | ND | ND |

| | | | | |
|---|---|---|---|---|
| +: 90% or more of strains are positive | | | | |
| -: 10% or less of strains are positive | | | | |
| D: dependent on species | | | | |
| ND: Not determined | | | | |

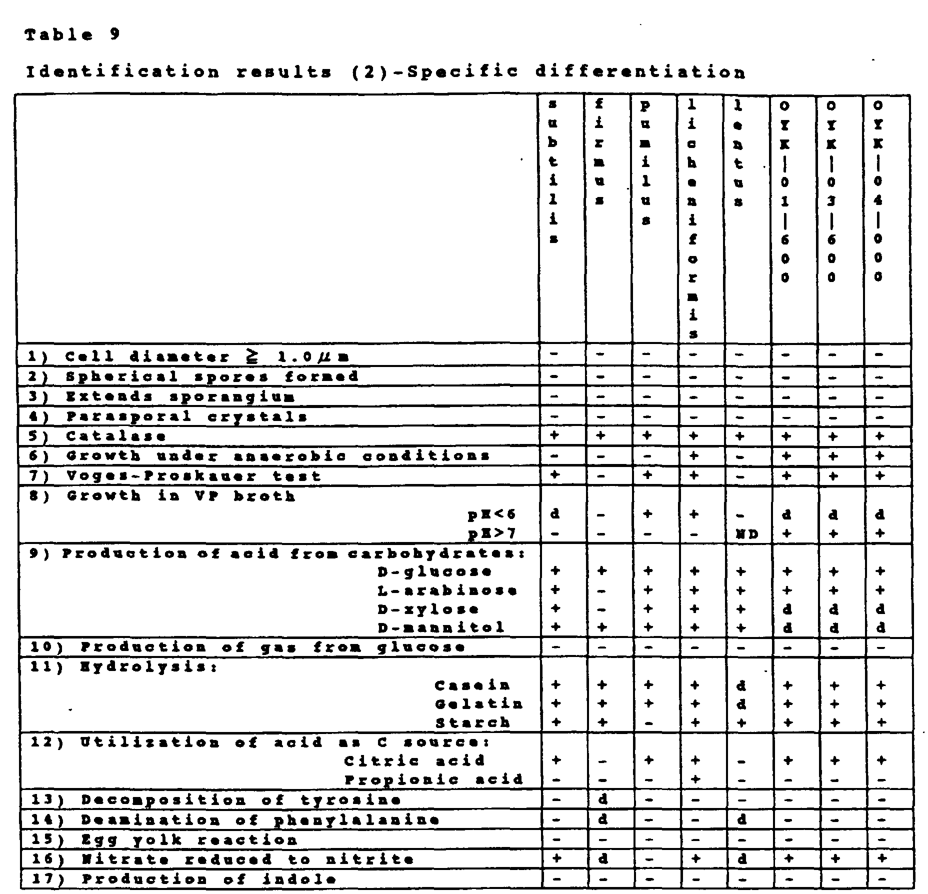

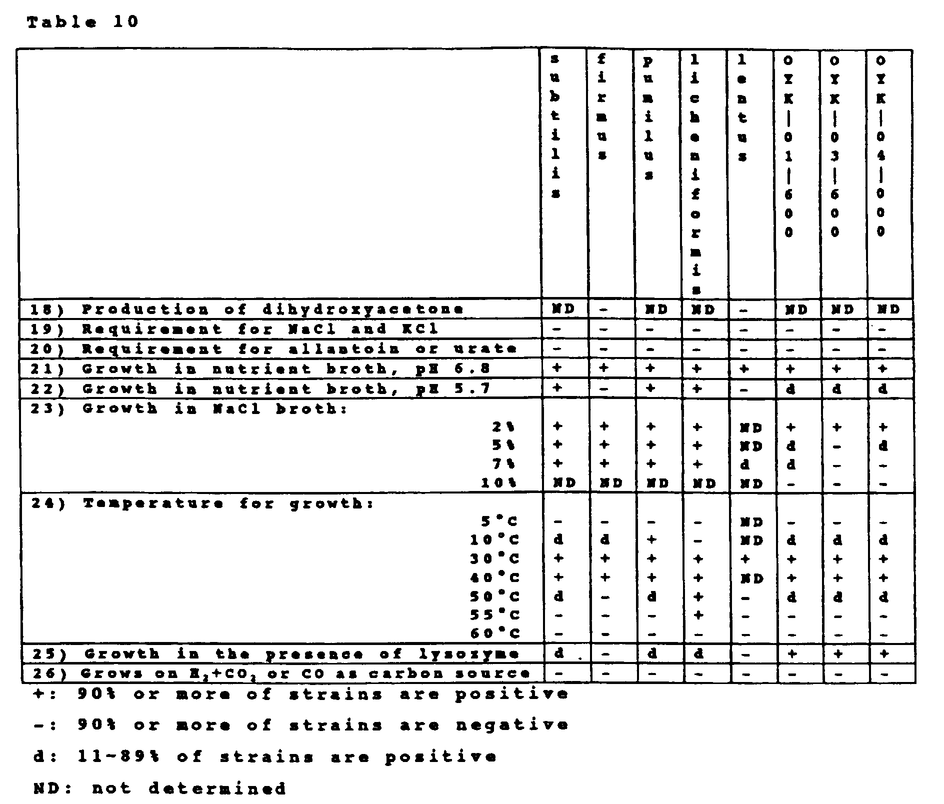

From Table 8, the above three strains are invariably considered to belong to the genus Bacillus, and from Tables 9 and 10, they are considered to be organisms related to B. subtilis. However, in view of the fact that they grow under anaerobic conditions, that the production of acid from carbohydrates is weak, and that they do not grow in 7% NaCl broth, they could not be identified with B. subtilis but were considered to be neotypes and each designated as Bacillus sp.

The above mutant strains are active against microorganisms the growth of which is accompanied by evolution of noxious odors. Moreover, the antimicrobial activity involved can be isolated and purified from cultures of the above strains. The antimicrobial substance so isolated and purified can be applied, either as it is or as dissolved or dispersed in a suitable solvent, e.g. water, optionally after dilution to a suitable concentration or admixing with other substances in advance, to substrates which are to be protected from adventitious microorganisms or made odorless or substrates to be provided with such properties, by using at least one means selected from among dusting, coating, impregnation, dipping, engaging, bonding, deposition, incorporation, addition, oral feeding and injection; said substrates including textile products, bedding, clothing, medical devices, sanitary equipment and fixtures, sanitary goods, footwear, filters for various air conditioning equipment and machines (inclusive of air cleaners), interior and exterior building materials, furniture, animals, animal quarters, animal husbandry systems and devices, water and feeds for animal breeding, dinnerware, chemical products, effluents or effluent treatment facilities and plants, microcapsules, passenger cars, ship or aircraft interior materials inclusive of upholstery, and morgue and funeral supplies, among others.

Further explanations on this subject are given in the working examples which appear hereinafter. C. Confirmation of antimicrobial activity of the microorganisms (Results are shown below in Table 11)

It was confirmed by the following experiments that Bacillus sp. OYK-01-600 (FERM BP-6394), Bacillus sp. OYK-03-600 (FERM BP-6395), and Bacillus sp. OYK-04-000 (FERM BP-6396) secrete antimicrobial activity.

### 1. Preparation of microbial suspensions

### (1) Strains

The three strains of OYK-01-600, OYK-03-600 and OYK-04-000.

### (2) Preculture

### [Medium composition] (in 1,000 ml of distilled water)

Peptone 10 g, meat extract 5 g, sodium chloride 5 g, agar 10 g

### [Cultural conditions]

The medium is set in sterilized dishes and the stock culture inoculum is streak-cultured on the medium. 37°C × 1 day

### (3) Fermentation culture

### [Medium composition] (in 1,000 ml of distilled water)

Peptone 10 g, meat extract 5 g, sodium chloride 5 g

### [Cultural conditions]

The medium is dispensed into sterilized test tubes and a loopful of (2) is suspended in the medium. 37°C × 1 day, under shaking at 170 cycles/min.

### 2. Preparation of a suspension of the tester strain

### (1) Tester strains for antimicrobial activity assay:

Klebsiella pneumoniae ATCC 4352, Staphylococcus aureus ATCC 6538P.

### (2) Preculture

### [Medium composition] (in 1,000 ml of distilled water)

Calf brain infusion 200 g, bovine heart infusion 250 g, proteose peptone 10 g, glucose 2 g, sodium chloride 5 g, disodium hydrogenphosphate 2.5 g, agar 15 g

### [Cultural conditions]

The medium is set in sterilized dishes and the inoculum is streak-cultured on the medium. 37°C × 1 day

### (3) Fermentation culture

### [Medium composition] (in 1,000 ml of distilled water)

Peptone 10 g, meat extract 5 g, sodium chloride 5 g

### [Cultural conditions]

The medium is dispensed into sterilized test tubes and (2) is added and shake-cultured.
- Klebsiella pneumoniae: 37°C × 20 hr., 170 cycles/min.
- Staphylococcus aureus: 37°C × 10 hr., 170 cycles/min.

### (4) Adjustment of the bacterial count

Using sterile buffered physiological saline, the viable count is adjusted to 5∼30×10⁵ cells/ml.

### 3. Antimicrobial activity assay

### (1) Preparation of test media

1) The suspensions of 2 tester organisms prepared in (4), 0.2 ml each, are respectively diluted with a medium composed of peptone 10 g, meat extract 5 g, sodium chloride 5 g and agar 10 g (in 1,000 ml of distilled water) and set in sterilized dishes.
2) The medium composed of peptone 10 g, meat extract 5 g, sodium chloride 5 g and agar 10 g (in 1,000 ml of distilled water) was set in sterilized dishes, and using a spreader, 0.2 ml of the suspension of each tester organism prepared in (4) is inoculated over the entire surface.

### (2) Preparation of reference microorganisms to be compared with the present strains for antimicrobial activity

For comparison with the 3 test strains of OYK-01-600, OYK-03-600 and OYK-04-000, 3 Bacillus subtilis strains inclusive of B. natto, designated A, B and C, were provided.

### (3) Inoculation with the test strains and reference strains

The 3 test strains of OYK-01-600, OYK-03-600 and OYK-04-000 mentioned under (3) in "1. Preparation of Microbial Suspensions" above and the 3 reference strains mentioned under (2) above, or a total of 6 strains, were used to inoculate the test medium prepared under (1) by the following procedures 1) and 2).
1) A penicillin cup is set in the center of each dish and 0.05 ml of the microbial suspension is poured into the cup.
2) A 0.02 ml portion of each microbial suspension is dripped in the center of each dish. The inoculated dish is incubated under stationary conditions at 37°C × 1 day.

### (4) Evaluation criteria

- ++: No growth of the tester strain beneath or adjacent to the colony of the test organism but an inhibition zone (halo) of not less than 2 mm is observed.
- +: No growth of the tester strain beneath or adjacent to the colony of the test organism but an inhibition zone (halo) of 0∼2 mm is observed.
- +-: No inhibition zone (halo) is observed but there is no growth of the tester strain within the colony of the test organism.
- -: Growth of the tester strain is found locally in the colony of the test organism.
- --: Growth of the tester strain is found locally in the colony of the test organism.
- ND: Not determined.

**Table 11**

| Results of assay of the antimicrobial activity processed by the microorganisms | | | | | | | | | |
|---|---|---|---|---|---|---|---|---|---|
| Tester strain | | Klebsiella pneumoniae | | | | Staphylococcus aureus | | | |
| Method for preparation of test medium | | Surface Smearing | | Spread-plating dilution | | Surface spreading | | Mix-dilution | |
| Method for inoculation with test organism | | Cup | Drip | Cup | Drip | Cup | Drip | Cup | Drip |
| Test organism | Ba. sp. OYK-01 | + | ++ | + | + | ++ | ++ | + | ++ |
| | Ba. sp. OYK-03 | + | ++ | + | + | ++ | ++ | + | ++ |
| | Ba. sp. OYK-04 | ++ | ++ | + | + | ++ | ++ | ++ | ++ |
| | Ba. sp. A | -- | -- | -- | -- | - | -- | -- | - |
| | Ba. sp. B | -- | -- | ND | ND | -- | -- | -- | -- |
| | Ba. sp. C | -- | -- | ND | ND | -- | -- | -- | -- |

As shown in Table 11, the test microorganisms in the growth phase secrete a substance active against both of Klebsiella pneumoniae and Staphylococcus aureus. D. Test for deodorization of an effluent treatment facility with the microorganisms (Results are shown below in Table 12)

It was confirmed by the following experimentation that feeding of Bacillus sp. OYK-01-600 (FERM BP-6394), Bacillus sp. OYK-03-600 (FERM BP-6395) or Bacillus sp. OYK-04-000 (FERM BP-6396) into an effluent treatment tank is effective in deodorizing the effluent treatment facility environment.

### 1. Summary of the test effluent treatment facility

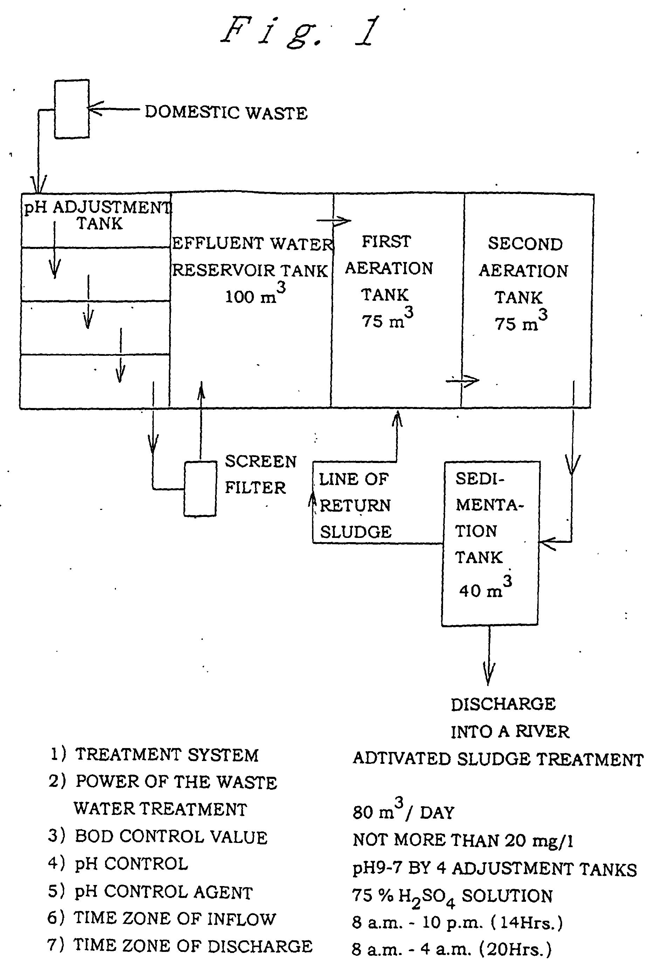

(1) Location and classification of business Mie Prefecture, laundry industry
(2) Effluent treatment plant and capacity Fig. 1

### 2. Protocol for deodorization test

### (1) Preparation of a microbial suspension

For this test, Bacillus sp. OYK-01-600 (FERM BP-6394) was chosen and a bacterial suspension was prepared as described under 1. Preparation of Microbial Suspensions.

### (2) Feeding the microorganism to the effluent treatment plant

The bacterial suspension prepared under (1), 2.5 liters, was fed to the effluent water reservoir tank of the effluent treatment plant illustrated in Fig. 1.

### (3) Evaluation of the effect

1) Assessors: 3 persons employed in the above facility
2) Assessment criteria: a 6-grade odor intensity rating method

### Intensity of odor:

0 = no odor
1 = a scarcely perceptible odor (detection threshold concentration)
2 = a mild odor suggestive of the source of odor (identification threshold concentration)
3 = a readily perceptible odor
4 = an intense odor
5 = a very intense odor

### 3) Location of assessment

The location where the active sludge from the sedimentation tank is recycled to the aeration tank.

### 4) Other parameters measured

BOD, the number of bacterial cells per mol of the feed

**Table 12**

| Results of a deodorization test in an effluent treatment facility | | | | | | | | | |
|---|---|---|---|---|---|---|---|---|---|
| Feeding to effluent reservoir tank | | Immediately after feeding | | After 1 month | | After 2 months | | After 3 months | |
| Frequency of odor intensity assessment | | 1 | 2 | 1 | 2 | 1 | 2 | 1 | 2 |
| Assessor | A | 5 | 5 | 3 | 3 | 1 | 1 | 0 | 0 |
| | B | 5 | 5 | 3 | 2 | 2 | 1 | 0 | 1 |
| | C | 5 | 4 | 2 | 1 | 0 | 0 | 0 | 0 |
| BOD (mg/l) | | 16 | | 14 | | 14 | | 18 | |
| Bacterial count (cells/ml) | | 10⁰ | | 10² | | 10⁴ | | 10⁵ | |

As shown in Table 12, it was confirmed that the intensity of odor declines with proliferation of the microorganism. This is suspected to be partly because the growth of this microorganism is scarcely accompanied by production of hydrogen sulfide, methylmercaptan, triethylmethane, etc. which are unpleasant for humans even at low concentrations and partly because the glycolysis does not involve evolution of gas. It has not been established as yet whether, when other microbial life proliferates, this microorganism vegetatively consumes the gas evolved.

It was confirmed that similar satisfactory results can be obtained with Bacillus sp. OYK-03-600 (FERM BP-6395) and Bacillus sp. OYK-04-000 (FERM BP-6396) as well.

### E. Isolation and purification of substances secreted by the microorganisms

The substances secreted by Bacillus sp. OYK-01-600 (FERM BP-6394), Bacillus sp. OYK-03-600 (FERM BP-6395) and Bacillus sp. OYK-04-000 (FERM BP-6396) and showing antimicrobial activity against Klebsiella pneumoniae and Staphylococcus aureus, were isolated and purified in the following manner.

### 1. Preparation of microbial suspensions

### (1) Microorganism

The 3 strains of OYK-01-600, OYK-03-600 and OYK-04-000 and the 3 strains of Bacillus subtilis inclusive of B. natto, namely strains A, B and C, procedure, were subjected to the same treatment.

### (2) Preculture

### [Medium composition] (in 1,000 ml of distilled water)

Peptone 10 g, meat extract 5 g, sodium chloride 5 g, agar 10 g

### [Cultural conditions]

The medium is set in sterilized dishes and streaked with the stock culture. 37°C × 1 day.

### (3) Fermentation culture

### [Medium composition] (in 1,000 ml of distilled water)

Peptone 10 g, meat extract 5 g, sodium chloride 5 g

### [Cultural conditions]

A sterilized Sakaguchi flask is charged with the medium and a loopful of (2) is suspended in the medium. 37°C × 2 days, under shaking at 170 cycles/min.

### 2. Separation of cells and secretions

The culture broth obtained is centrifuged with a refrigerated (4°C) centrifugal machine at 12,000 RPM for 14 minutes. The supernatant alone is withdrawn and suction-filtered through a 0.45 µm membrane filter.

### 3. Concentration of secretions

The filtrate is transferred to an egg plant-shaped flask and the filtrate is thoroughly frozen on the flask wall and, then, dehydrated with a vacuum freeze-dryer for 48 hours to provide a purified powder.

### F. Antimicrobial activity test of the substances secreted by the microorganisms (Results are shown below in Table 13)

The activity of the purified substances suspected to have antimicrobial activity as isolated from secretions of Bacillus sp. OYK-01-600 (FERM BP-6394), Bacillus sp. OYK-03-600 (FERM BP-6395) and Bacillus sp. OYK-04-000 (FERM BP-6396) was assayed by the procedure described below.

### 1. Test substances

The 6 substances obtained by the procedure described under 3. Concentration of Secretions:
(1) Bacillus sp. OYK-01-600 (FERM BP-6394)
(2) Bacillus sp. OYK-03-600 (FERM BP-6395)
(3) Bacillus sp. OYK-04-000 (FERM BP-6396)
(4) Bacillus sp. A
(5) Bacillus sp. B
(6) Bacillus sp. C
   and the following antimicrobial activity reference control substances (7) and (8), or a total of 8 substances.
(7) Chloramphenicol (an antibiotic manufactured by Wako Pure Chemical Ind. Co., Ltd.; Mw: 323.13, CAS: 56-75-7, which has protein synthesis-inhibitory activity and is active against gram-positive and gram-negative bacteria, rickettsia and viruses. Its antimicrobial activity is usually bacteriostatic. In the antimicrobial activity test, 0.2 ml of an aqueous solution of 10 mg/100 ml concentration is used).
(8) Nikkanon RB (a quaternary ammonium synthetic antimicrobial agent manufactured by Nikka Kagaku Kogyo K.K.; this substance has high antibacterial activity against Staphylococcus aureus and antifungal activity as well. A laundry-resistant antimicrobial deodorant. In the antimicrobial activity test, 0.2 ml of an aqueous solution of 1 ml/100 ml concentration is used). Thus, a total of 8 test substances, (1)∼(8), are used.

### 2. Tester strains for antimicrobial assay

The two strains mentioned in "2. Preparation of Tester Strain Suspensions" described above.
(1) Klebsiella pneumoniae ATCC 4352
(2) Staphylococcus aureus ATCC 6538P

### 3. Protocols for antimicrobial activity tests

### (1) Method for assay according to the degree of turbidity of a culture

### 1) [Medium composition] (commercial nutrient broth, in 1,000 ml of distilled water)

Meat extract 5 g, peptone 10 g, sodium chloride 5 g

### 2) [Cultural conditions]

The medium is dispensed, in 5 ml aliquots, into sterilized test tubes and 0.02 g of the test substance and 0.1 ml of the tester strain suspension are added and mixed. Shake culture at 37°C × 1 day, 170 cycles/min.

### 3) [Observation parameter]

The transmittance of the culture is measured with the Model 100-20 spectrophotometer manufactured by Hitachi, Ltd. The measuring wavelength: 415 nm, the indication unit: % (the population of about 5∼30×10⁸ cells of Klebsiella pneumoniae gives a transmittance of 52%).

### (2) Method for assay according to the number of bacterial cells

### 1) [Medium composition] (commercial broth agar medium, in 1,000 ml of distilled water)

Meat extract 5 g, peptone 10 g, sodium chloride 5 g, agar 15 g

### 2) [Cultural conditions]

The medium is dispensed, in 15 ml aliquots, into sterilized dishes and 0.02 g of the test substance is mixed therein, followed by solidification. Then, 0.2 ml of the tester strain suspension is smeared evenly on the surface. 37°C × 1 day.

### 3) [Observation parameter]

The colonies grown on the dish are counted. The indication unit: the exponent (e.g. "B" in the case of A×10^{B})

### (3) Method for assay according to inhibition zone (halo)

### 1) [Medium composition] (commercial broth agar medium, in 1,000 ml of distilled water)

Meat extract 5 g, peptone 10 g, sodium chloride 5 g, agar 15 g

### 2) [Cultural conditions]

The medium is dispensed, in 15 ml aliquots, into sterilized dishes and 0.2 ml of the tester strain suspension is evenly spread on the surface. After the surface has become semi-dry, 0.02g of the test substance in solid form is placed in the center of the dish and stationary culture is carried out. 37°C × 1 day.

### 3) [Observation parameter]

The maximum diameter of the halo formed is measured. Indication unit: mm

### (4) Penicillin cup assay

### 1) [Medium composition] (commercial nutrient broth, in 1,000 ml of distilled water)

Bottom layer: meat extract 5 g, peptone 10 g, sodium chloride 5 g, agar 15 g
Top overlayer: meat extract 5 g, peptone 10 g, sodium chloride 5 g, agar 7 g

### 2) [Cultural conditions]

The bottom-layer medium is dispensed in 15 ml aliquots into sterilized dishes and solidified. On top of this, a mixture of 4 ml of the top-layer medium and 0.1 ml of the tester strain suspension is overlayed. After the surface has become semi-dry, a stainless steel cup (6 mm inside dia., 8 mm outside dia., 10 mm high) is dropped perpendicularly onto the center of the dish from a height of 10∼13 mm. The cylinder is filled with 0.02 g of the test sample dissolved in 0.2 ml of sterile distilled water and stationary culture is performed. 37°C × 1 day.

### 3) [Observation parameter]

Growth of the tester stain in and out of the cup is observed.

### Indication unit:

- --: Growth of the organism in the whole cup
- +-: Growth of the organism in a portion of the cup
- +: No growth of the organism in the cup
- A: A halo with a maximum diameter of A mm (an example) is observed externally of the cup

### [Reference to the deposited biological materials]

(1) Name of the deposit organization: Patent Microorganism Deposit Center, National Institute of Bioscience and Human Technology, Agency of Industrial Science and Technology, MITI, Japan.
   Address: Postal zone 305-0046
   Higashi 1-1-3, Tsukuba-shi, Ibaraki, Japan (Tel. 0298-54-6029)
(2) Date of deposit: June 29, 1998
(3) Accession Nos.:
   1) FERM BP-6394
   2) FERM BP-6395
   3) FERM BP-6396

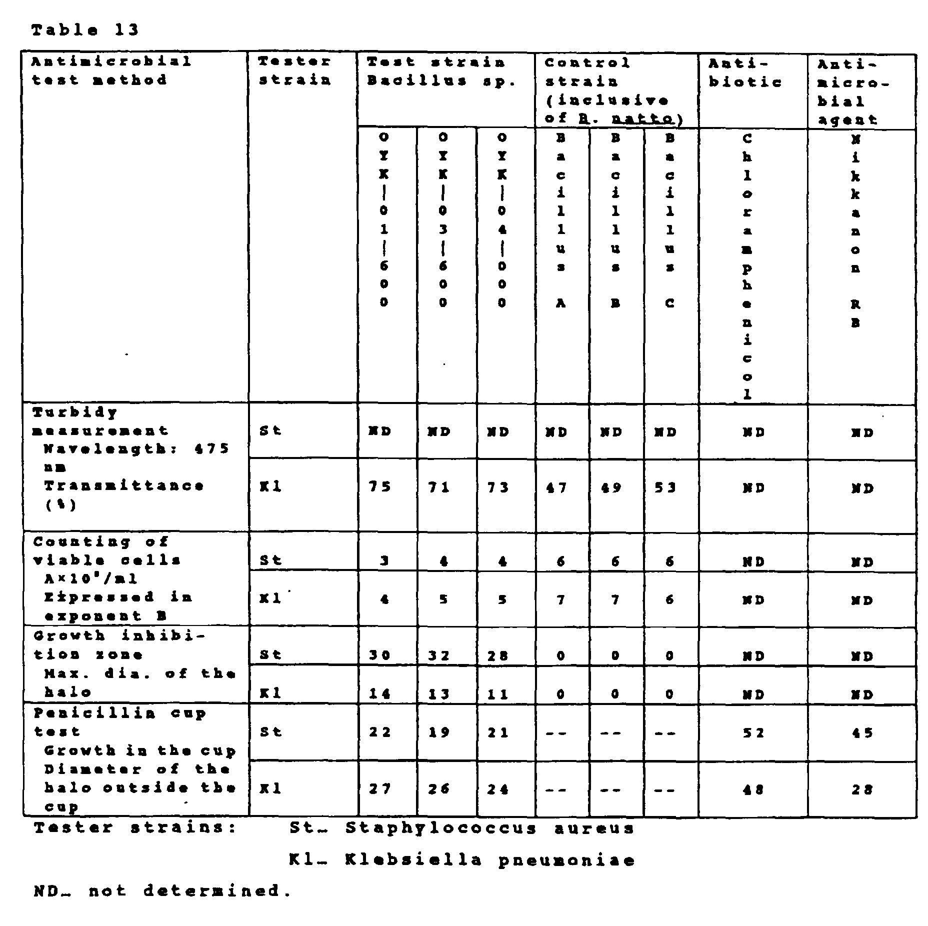

As shown in Table 13, the substance secreted by the present strains of microorganism have antimicrobial effects.

### G. The antimicrobial activity test of the products of OYK strains

As the OYK microorganism, Bacillus sp. OYK-01-600 was used but similar results were obtained with Bacillus sp. OYK-03-600 (FERM BP-6395) and Bacillus sp. OYK-04-000 (FERM BP-6396) as well.

### 1. Isolation and purification of the elaboration products of the OYK microorganism

### [1] Extracellular product

(1) The stock culture stored frozen at -82°C, 1 ml, was thawed.
(2) A test tube was charged with 4 ml of sterilized BGG liquid medium (a medium composed of soybean protein extract, glucose and sodium glutamate; the same applies hereinafter) and the bacterial preparation obtained in (1) above was inoculated therein and seed culture was carried out at 37°C for 24 hours.
(3) A Sakaguchi flask was charged with 100 ml of sterilized BGG liquid medium and the organism cultured in (2) was added and fermentation culture was carried out at 37°C for 72 hours. (OD=10, pH=7.9)
(4) The culture was then centrifuged and the supernatant was recovered. (12,000 rpm × 10 min, 4°C)
(5) The supernatant (4) was filtered through a membrane filter (0.2 µm filter).
(6) The filtrate (5) was freeze-dried.
(7) The freeze-dried preparation obtained in (6) above was dissolved in 3∼4 ml of physiological saline.
(8) In the antimicrobial test described below, 50 µ l of the solution obtained in (7) above was dripped.

### (2) Intracellular product

(1) The stock culture stored frozen at -82°C, 1 ml, was thawed.
(2) A test tube was charged with 4 ml of sterilized BGG liquid medium and the medium was inoculated with the above stock culture (1). Seed culture was performed at 37°C for 24 hours.
(3) A Sakaguchi flask was charged with 100 ml of sterilized BGG liquid medium and the medium was inoculated with the above culture (2). Fermentation culture was performed at 37°C for 72 hours. (OD=10, pH=7.9)
(4) The resulting culture was centrifuged and the pellet was recovered. (12,000 rpm × 10 min., 4°C)
(5) The above pellet was dispersed by adding 5 ml of physiological saline.
(6) To the pellet dispersion was added 20 ml of n-butanol, and the mixture was stirred for 1 hour and then allowed to stand, whereupon an aqueous layer separated from an alcohol layer.
(7) The alcohol layer obtained in (6) above was recovered and the n-butanol was removed by evaporation.
(8) The residue was transferred to a desiccator for further dehydration.
(9) The dry purified product thus obtained was dispersed in 1 ml of physiological saline.
(10) The dispersion was filtered through a membrane filter. (0.2 µm filter)
(11) In the following antimicrobial activity test, 50 µl of the filtrate obtained in (10) above was added by dripping.

### 2. Antimicrobial activity assay of the fermentation products of the OYK organisms

### [1] Test fermentation products

(1) The extracellular product of the OYK organism as obtained in 1-[1].
(2) The intracellular product of the OYK organism as obtained in 1-[2].

### [2] Tester strains

(1) Staphylococcus aureus (ATCC25923)
(2) Klebsiella pneumoniae (ATCC13883)
(3) MRSA1002 (a clinical isolate of meticillin-resistant Staphylococcus aureus)
(4) Escherichia coli O-157 (S180)
(5) Legionella pneumophilla (OCI 88171)
(6) Pseudomonas aeruginosa (IFO3452 (red pigments))
(7) Pseudomonas aeruginosa (ATCC27853 (blue pigments))
(8) Fuzarium proliferatum Nirenberg S12 (IFO6349)
(9) Aspergillus niger van Tieghem S1 (IFO6341)
(10) Penicillium cittrinum Thom S5 (IFO6352)
(11) Trichophyton mentagrophytes (IFO5466)
(12) Rhizopus stolonifer Lind S7 (FERM S-7)

### [3] Test protocol

(1) All the test organisms and tester strains were respectively seed-cultured under optimum conditions;
(2) The suspension of each tester strain, which contained a predetermined number of cells, was smeared in a given inoculum size on the optimum solid medium for the same tester strain and the inoculated medium was allowed to stand until it had become semi-dry.
(3) Each of the two test fermentation products (see [1] above), 50 µl, was dripped on the medium (2) above.
(4) Culture was performed under the optimum conditions for growth of each tester strain.
(5) Formation of a growth inhibition zone (halo) about the drip point was used as an indicator of antimicrobial activity. The diameters (maximum and minimum diameters) of the inhibition zone were measured (unit: cm). The results are shown below in Table 14.

**Table 14**

| | Fermentation products of OYK organism | |
|---|---|---|
| | Extracelluar product | Intracelluar product |
| (1) Staphylococcus aureus | 2.04×1.92 | 1.20×1.20 |
| (2) Klebsiella pneumoniae | 0.84×0.96 | 1.81×1.81 |
| (3) MRSA1002 | 1.32×1.63 | 2.04×1.20 |
| (4) Escherichia coli O-157 | 1.20×1.50 | 2.04×1.69 |
| (5) Legionella pneumophilla | 1.30×1.00 | 1.60×1.30 |
| (6) Pseudomonas aeruginosa (red pigments) | 1.68×1.68 | -- |
| (7) Pseudomonas aeruginosa (blue pigments) | 1.56×1.20 | 2.40×3.24 |
| (8) Fuzarium proliferatum | 1.20×1.20 | -- |
| (9) Aspergillus niger | 1.92×1.92 | -- |
| (10) Penicillium cittrinum | 2.40×2.76 | -- |
| (11) Trichophyton mentagrophytes | 0.96×1.20 | -- |
| (12) Rhizopus stolonifer | 2.40×2.16 | -- |

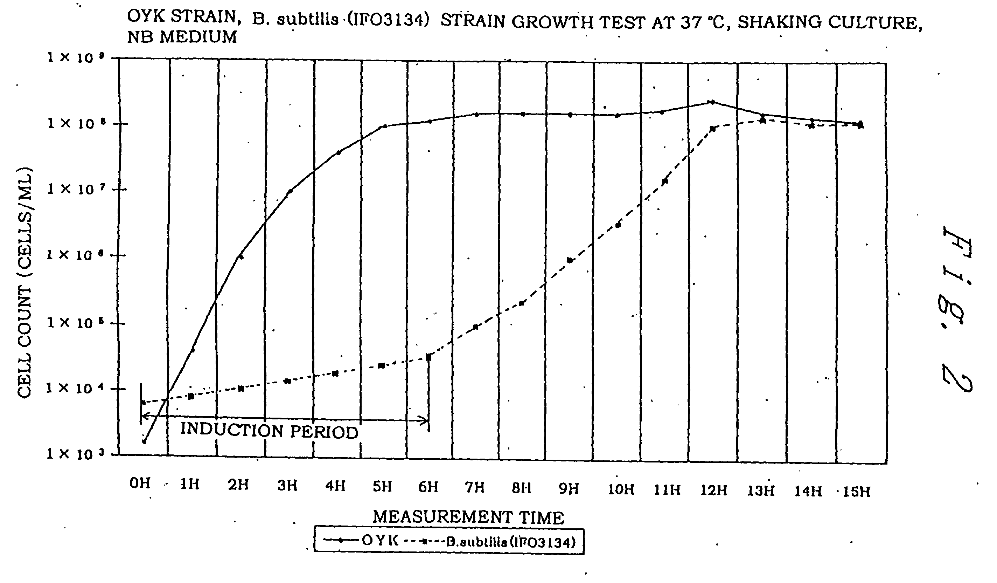

### H. Growth velocity test with the OYK organism (Fig. 2)

The novel microorganism of the present invention begins to grow quickly after the start of culture without requiring an induction period (acclimatization period for proliferation). For example, under the cultural conditions of 20∼40°C, the initial concentration of about 10³ cells/ml (g) increases to not less than 10⁸ cells/ml (g) within 6 hours.

Fig. 2 shows the time course of growth of Bacillus sp. OYK-01-600. As control, the time course of growth of the type culture of B. subtilis (IFO 3134) is also shown. The growth curves in Fig. 2 were constructed by plotting the logarithm of the number of bacterial cells on the ordinate against time on the abscissa. It will be apparent if only from the graph that the OYK strain of the invention has substantially no induction period but begins to multiply rapidly, with the initial concentration of about 10³ cells/g reaching a concentration of 10⁸ cells/ml or more in about 5 hours. In contrast, the type culture of B. subtilis (IFO 3134) begins to grow after an induction period of 6-8 hours following the start of culture and it took about 12 hours for the organism to multiply to the concentration of about 10⁸ cells/g. On the other hand, the OYK strain is 4 times as large in volume as said type culture strain, and whereas the volume multiple of the type culture strain was 6 after 4 hours, that of the OYK strain was 20,000. Thus, in terms of volume, the magnitude of increase was 20,000 × 4 = 80,000 times. By simple comparison, the volume occupied by the present strain is 80,000 ÷ 6 = 13,333..., or more than 10,000 times as large.

### I. Utilization of the OYK organism

While Bacillus sp. OYK-01-600 was used as the OYK strain, similar satisfactory results were obtained with Bacillus sp. OYK-03-600 (FERM BP-6395) and Bacillus sp. OYK-04-000 (FERM BP-9364) as well.

### (Preparation of the OYK powder)

The process for preparing a powder of the OYK organism is described below.
(1) The stock culture stored frozen at -82°C, 1 ml, was thawed.
(2) Sterilized BGG liquid medium, 4 ml, was taken in a test tube, the thawed organism (1) was then placed in the tube, and seed culture was carried out at 37°C for 24 hours.
(3) Sterilized BGG liquid medium, 100 ml, was taken in a Sakaguchi flask, the above culture (2) was placed therein, and fermentation culture was carried out at 37°C for 24 hours. The OD value at this stage was about 10, and the number of cells was 1×10⁸ cells/ml.
(4) The broth obtained in (3) was lyophilized to give 1 g of a bacterial powder (containing the medium). The number of cells was 1×10¹⁰.

In the bacterial powder thus obtained, the OYK organism had been sporulated. This condition leads to the following beneficial results.
[1] Because of sporulation, the organism can be stored at ambient temperature without risks for a decrease in population or mutation, only if provision is made for prevention of moisture.
[2] The powder can be compression-molded with a tablet machine at a punching pressure of about 10 tons. There is no compression-associated decrease in cell count.
[3] Chemicals necessary for tablet production, such as a perfume, a coagulant, a dissolution retardant, etc., can be added.

### (Utilization as tablets)

The bacterial powder obtained in the above example was supplemented with additives for promoting bacterial growth (e.g. glucose, sodium glutamate, soybean protein extract, culture medium components, etc.) and compressed with a tablet machine to provide tablets. When the tablets were added to a garbage, the malodor emanating from the garbage was successfully eliminated. The supposed mechanism for this outcome is that the tablets put in the garbage are dissolved by the available moisture and water and the spores supplied with water begin to act as living OYK organisms and multiplied at a speed surpassing the rate of growth of odor-producing putrefactive bacteria. Since the OYK strain shows antimicrobial activity against E. coli O-157, the tablets can be used in the kitchen for prevention of food poisoning.

### (Example of production of a compost and the effect of the compost)

Furthermore, the OYK strain of the invention expresses a useful action in the course of production of a compost. Particularly in the compost production process, the organism exerts a useful effect particularly in the initial rise-up of fermentation temperature. A relevant example is described below.

The number of days required for 700 g of bovine manure to reach a temperature of 50°C was 8 days. In contrast, when 700 g of bovine manure containing 50 ml of a 1×10⁸ cells/ml suspension of OYK strain (OYK-01-600) added was similarly tested, it took only 3 days, showing a shortening of growth time. As to the duration of warmth retention at 40°C or higher, whereas it was 6 days for the untreated manure, the duration for the manure containing the OYK strain added was 17 days, indicating a prolongation of warmth retention time. It is said that in the process of compost formation, many bacteria and fungi repeat multiplication and death in respective temperature ranges to thereby reduce the molecular weight of high molecular organic matter. In this cycle, the strain survives the maximum temperature of 70°C in the form of spores and proliferate activity in the neighborhood of 40°C in the temperature rise and fall phases whereby the required number of cycles is reduced. Moreover, as the OYK strain as its characteristic does not produce a malodor in the course of its growth, the emanation of mercaptan and hydrogen sulfide odors during compost formation was inhibited.

### (Preparation of the OYK organism as a sheet and its utilization)

An attempt was made to expediently utilize the effect of the OYK organism of the invention in the field of health care. This attempt is now described in detail.

A 1×10⁷ cells/ml suspension of the strain (OYK-01-600) was mixed with 10% of the binder PVA (polyvinyl alcohol) and the resulting dispersion was used to print a nonwoven cloth weighing 100 g/m² using a 1500-mesh screen printing press. After evaporation of water, the nonwoven cloth was examined under the microscope. It was confirmed that the OYK organisms had been sporulated and immobilized as intertwined with the filaments of the nonwoven cloth.

This nonwoven cloth was cut to a suitable size and spread on the bed sheet so that it could be applied against the back of a patient having a bed sore of which Pseudomanas aeruginosa had been identified to be the cause. On observation, the malodor disappeared progressively and a remission of the bed sore could be confirmed.

While a nonwoven cloth was used in the above example, this is not an exclusive choice but a woven or knit cloth made of synthetic or natural fiber, a synthetic resin sheet or a foamed synthetic resin sheet can also be employed.

### (Utilization for purification of pond water)

The ponds in the golf course are in eutrophicated state because the fertilizer applied to the turf in large quantities is leached out. In such ponds, reddish phytoplanktons undergo abnormal proliferation at times and this growth leads to decreases in dissolved oxygen in the pond water. As a consequence, anaerobic bacteria are encouraged to grow, causing evolution of odoriferous gases. When 10 liters of a suspension of the OYK bacteria (OYK-01-600, concentration 1×10⁸ cells/ml) was poured in a pond presenting with such manifestations (quantity of water of the order of 400 t), the reddish color of the water surface disappeared and regained much of clarity in 2 weeks. The underlying mechanism has not been clarified as yet but it may be suggested that growth of the OYK bacteria deproves the pond water of nutrients and hence causes death of the phytoplanktons.

### (Utilization as a deodorant)

The water purifying effect on a eutrophicated pond has been described just above and the deodorant effect on effluent (waste water) treatment premises has been described in "D. Deodorization of An Effluent Treatment Facility with the Microorganism". For the control of malodors in the barns and yards for dairy cow or beef cattle, on poultry farms or hog-raising farms, the preventive effect of OYK bacteria against the emanation of mercaptan and hydrogen sulfide odors as described in the example of compost production suggests the possibility of deodorizing these facilities by application of OYK bacteria. In connection with slaughter houses, hospital operating theaters and sanitary filth disposal facilities, and animal hides and skins in leather manufacture, the OYK bacteria grow faster than the bacteria producing malodors in digesting protein, fat and carbohydrate to achieve a deodorization effect as can be seen from Table 12 and the paragraph immediately following the table. The OYK bacteria to be dusted, incorporated or deposited may for example be a bacterial suspension as such or a powder of the spores, and similar effects can be obtained with an intracellular secretion product or an extracellular secretion product of the bacteria.

### (Utilization for compost formation [I])

To ascertain the effect of OYK bacteria on the fermentation temperature, a compost-making experiment using small amounts of bovine manure was performed under interior conditions (room temperature 18°C). The details of the samples used are as follows.
- (1-1):
- Bovine manure: 400 g (water content 60%)
- CFP: 40 g (coffee grounds, moisture content 8%)
- Powdered bean curd refuse: 20 g (soybean protein source)
- OYK bacterial powder: 4 g (bacterial concentration 1×10¹⁰ cells/g)
- (1-2) Bovine manure: 400 g (water content 60%)

Each of the above samples was put in a thermos bottle and the time course of fermentation temperature was monitored. The results are shown in Fig. 3. Although the first day was assigned to preparation, the subsequent time course of temperature was monitored by measurements at 6-hour intervals. The curves in the figure are indicated by the corresponding sample numbers (1-1) and (1-2).

In Sample (1-1), the powdered bean curd refuse is an enrichment source contributory to growth of OYK bacteria and CFP is a porous substance having the property to adjust moisture, absorbs the ammonia odor produced in a minor amount and creates an aerobic environment. The OYK powder is a freeze-dried culture broth with a bacterial concentration of 1×10¹⁰ cells/g. In Sample (1-1), the temperature increased from room temperature to 70°C in about 1 day. The OYK bacteria were active over the range of 10∼55°C and most active in the range of 30∼40°C, elevating the manure temperature rapidly. Growth of OYK bacteria entailed production of ammonia in some amount and the resulting weak alkalinity coupled with the high temperature promoted the activity of actinomyces and other thermophiles. It is considered that these events raised the temperature of bovine manure to 70°C in only about one day as shown by the graph in Fig. 3.

The temperature fell to room temperature on the second day and subsequently but reshuffling entailed a re-elevation of fermentation temperature.

In Sample (1-2) on the other hand, the bovine manure temperature increased by about 10°C but the fermentation proceeded only insufficiently for compost formation.

In the above test, for accurate measurement of the fermentation temperature, the sample was placed in a thermos bottle and subjected to forced aeration.

### (Utilization in compost formation [II])

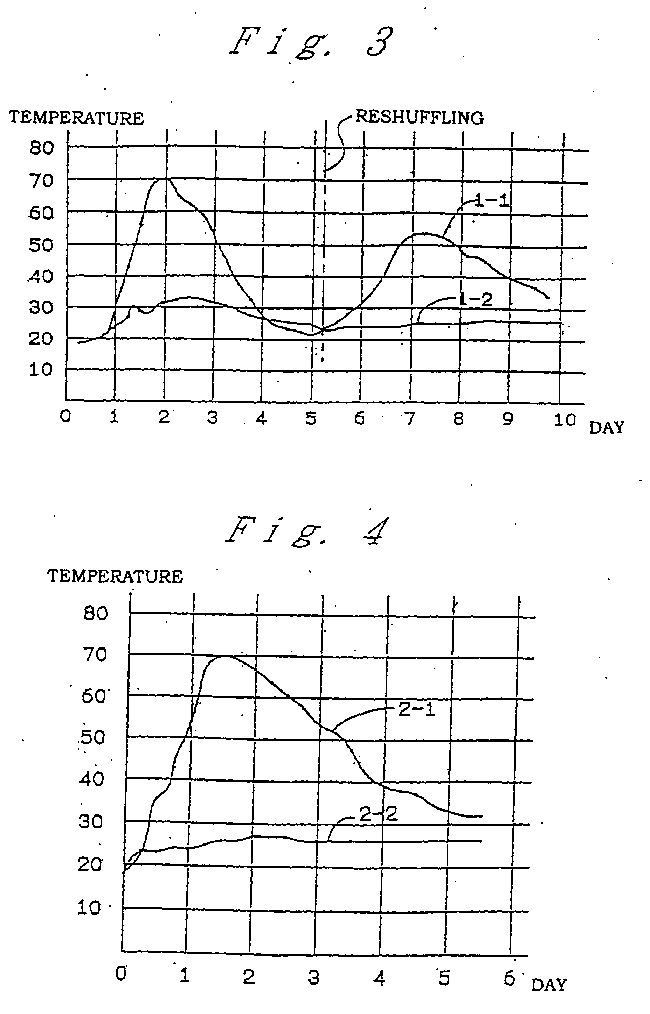

To investigate the influence of the moisture content of bovine manure, a similar test was performed using the following samples. The room temperature was 18°C. The time course of temperature of each sample is diagrammatically shown in Fig. 4.
- (2-1):
- Bovine manure: 400 g (not adjusted for water; moisture content 80%)
- CFP: 40 g (coffee grounds, moisture content 8%)
- OYK powder: 4 g (bacterial concentration 1×10¹⁰ cells/g)
- (2-2) Bovine manure: 400 g (moixture content 60%)

In Sample (2-1), the temperature continued to rise from room temperature to a peak of 70°C.

In Sample (2-2), the temperature rose only slightly and did not exceed 30°C.

### (Utilization in compost formation [III])

With reference to the results of the above examples 1∼2, an outdoor compost formation test was carried out. The volume was also increased to 7 t. The test location was a compost center in Shizuoka Prefecture and the test was carried out in winter when compost formation is normally difficult. The sample was piled up in bulk outdoors and covered with a sheet.
- (3-1):
- Bovine manure: 5 t (not adjusted for water; moisture content 80%)
- Recycled compost: 1.5 t (used for water adjustment; moisture content 44%)
- CFP: 150 kg (coffee grounds, moisture content 8%)
- Defatted soybean cake: 150 kg (soybean protein source)
- OYK powder: 5 kg (bacterial concentration 1×10¹⁰ cells/g)

The time course of fermentation temperature and that of outdoor atmospheric temperature are diagrammatically shown in Fig. 5. Because of the wintry season, the temperature of the sample was low and it was apprehended whether the fermentation temperature would rise, but actually an increase of 20°C was confirmed in one day, with the generation of vapor from the sample pile being observed. Thereafter, the temperature ran a time course similar to that found in the indoor test (described above), reaching a maximum level of 78°. Thereafter, the temperature fell so that "reshuffling" was performed. Then, a re-elevation of fermentation temperature took place.

### (Preparation of a bacterial cell preparation)

In the above examples, the microorganism was used directly in the form of a powder. However, an operation on a large scale requires a bacterial preparation suitable for efficient feeding of bacteria in a high concentration over a broad extent. An exemplary protocol for preparation of such a bacterial preparation is now described.
(1) The OYK strain stored frozen is inoculated into nutrient broth (meat extract medium) at a level of 1% and incubated at 37°C for 24 hours under shaking.
(2) Whole soybeans soaked in water for one day are sterilized by autoclaving at 121°C and 2 atm. for 20 minutes.
(3) The whole beans sterilized by autoclaving in step (2) are evenly spread in a flat vat.
(4) The whole beans are spray-inoculated with a suspension of OYK bacteria in a clean bench.
(5) The inoculated whole beans are left standing in a constant-temperature room at 37°C for 2 days and the number of bacteria is checked to confirm that the bacterial count is not less than 1×10⁸ cells/g.
(6) The batch which has undergone the above step (5) is thoroughly dried at 60°C and crushed into granules with a pulverizer. The OYK bacteria in this state are viable in sporulated form.

The granular product thus obtained is an example of the bacterial preparation according to the invention. While the dried batch is pulverized into granules in the above step (6), the diameter of the grain is adjusted within the range of fine powders to granules according to the kind of enrichment source used. This is because, with weight being kept constant, the finer the grain is, the greater is the number of growth points available to the bacteria.

### (Utilization of the bacterial preparation for compost formation)

To evaluate the field utility of the bacterial preparation of the invention, a compost formation experiment with the bacterial preparation was carried out in the fermentation lane of a compost center in Shizuoka Prefecture. This experiment was performed in a winter season unfavorable for compost formation.

The following samples were prepared.
- (4-1):
- Bovine manure: 54 t (not adjusted for water; moisture content 80%)
- CFP: 9 t (coffee grounds, moisture content 8%)
- Defatted soybean cake: 3 t (soybean protein source)
- OYK powder: 50 kg (a soybean protein-bacterial cell preparation)
- (4-2):
- Bovine manure: 54 t (adjusted for water)
- CFP: 9 t
- (4-3):
- Bovine manure: 54 t (not adjusted for water)

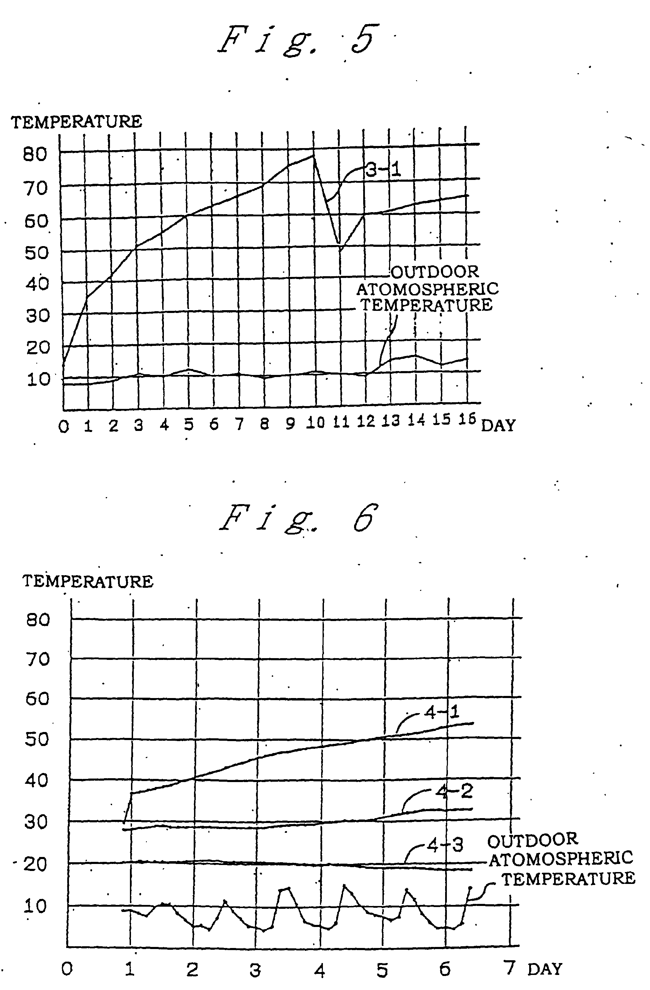

The time course of fermentation temperature and that of outdoor atmospheric temperature are diagrammatically shown in Fig. 6. The curves are represented by the numbers corresponding to the sample numbers. It can be seen that CFP as used in Sample (4-2) alone is not sufficiently contributory to the fermentation temperature and that in Sample (4-3) no appreciable elevation of fermentation temperature is obtained. It was thus demonstrated under field conditions that the bacterial preparation is remarkably effective in increasing the fermentation temperature. In Sample (4-1), the temperature began to rise immediately following commencement of the experiment and then continued to increase. The subsequent time course was substantially identical to that shown in Fig. 5, indicating the practical usefulness of the formulation.

The compost according to the above formula had no manure odor but a faint soil odor. The addition of CFP is effective in controlling moisture and retaining an aerobic environment and because the OYK strain, which is a facultative anaerobe, shows more active growth under aerobic conditions than under anaerobic conditions, CFP encouraged the activity of OYK bacteria. In compost formation in cold weather, the tendency of the temperature toward elevation owing to bacterial activity is quickly reversed by the deprivation of heat by the peripheral low-temperature zone so that the absolute increase in temperature is not more than 40°C in many instances. However, in the bovine manure containing OYK bacteria added, the OYK strain shows an abnormal growth about 10,000 times as great immediately after addition, causing an increase in temperature which cannot be cancelled by the peripheral low-temperature zone but rather the thermal energy diffusing into the peripheral zone where the viable organisms are encouraged to grow, thus elevating the temperature of the whole manure to a level close to 80°C. This temperature elevation is attributable to growth of thermophilic bacteria. In this manner, a safe full-ripe compost with the harmful microorganisms and weed seeds killed can be produced.

While the effect of the bacterial preparation of the invention has been described with bovine manure taken as the substrate in the foregoing examples, the preparation can, of course, be applied with equal success to excreta of other animals (e.g. poultry droppings and equine manure) and garbages.

### (Utilization of the bacterial preparation as a deodorant)

In 100 cc of water were dissolved 1.5 g of agar powder, 1 g of glucose, 1 g of sodium glutamate, 1 g of the soybean protein-bacterial cell preparation and 10 g of coffee grounds, and the solution was stirred sufficiently and dispensed into a vessel. Allowing the vessel to sit at 20°C for 1 day gave a solid composition.

This solid composition was administered to a kitchen sink drain port or a garbage basket. By this procedure, the emanation of malodors from the drain port and garbage basket could be prevented. The suspected mechanism is that the solid composition added dissolves gradually to release the OYK bacteria which, then, multiply to suppress growth of putrefactive bacteria and thereby arrest emanation of malodors.

### (The bacterial preparation in the tablet form)

The soybean protein-bacterial cell preparation, an aqueous solution of gelatin, and coffee grounds (CFP) were kneaded together and the water was evaporated off until a consistency free from disintegration had been obtained. Then, this kneaded mixture was compressed with a tablet machine to provide tablets. The gelatin served not only as a protein source for the bacteria but also as a binder effective for shape retention. CFP contributes to aerobicity.

In the tablets obtained, the bacteria are viable in the sporulated form. When the tablets encounter water, the bacteria begin to grow. Therefore, when the tablets are administered to a pond contaminated with organic matter, for instance, the tables conveniently float on the water surface and are disseminated. It is necessary that the bacteria should exist in a high concentration initially at administration and when the tablet is so designed that the bacteria will be disseminated over an increasingly broader range, the effect of the bacteria will be prominent and so broad as to cover the whole pond.

### (Preparation of a deodorant disk)

Production of a solid preparation in the form of a multi-perforated disk is now described.

In 100 cc of water were dissolved 1 g of agar powder, 1 g of glucose, 1 g of sodium glutamate, 1 g of soybean protein and 100 g of coffee grounds. This mixture was stirred thoroughly and autoclaved at 120°C for 20 minutes.

The above mixture is sufficiently fluid at a temperature of not less than 70°C and, therefore, the mixture (15) was poured into a plastic vessel (14) equipped with a plurality of upright projections (13) extending from its bottom surface and, then, allowed to cool down to ambient temperature.

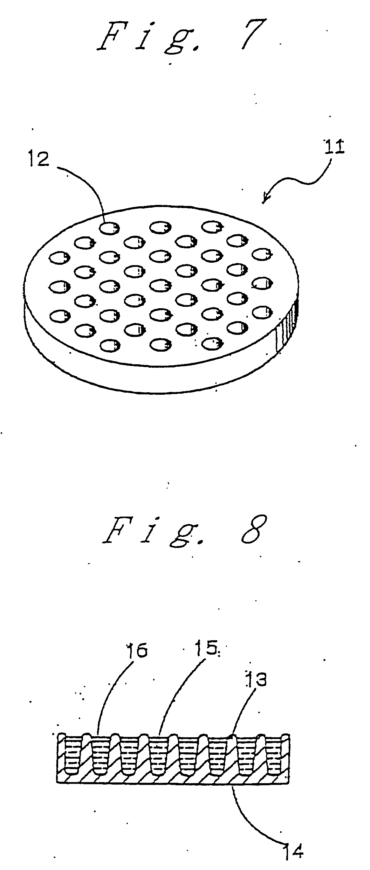

About 0.1 ml of a concentrated 1×10⁹ cells/ml suspension of the OYK strain (16) was sprayed over the mixture (15) and allowed to stand at an ambient temperature of 20°C for 24 hours, whereupon the mixture (15) was solidified. Because the mixture (15) solidified with reduction in volume, the solid mass could be easily released from the vessel (14) to provide a disk-shaped monolithic product (11) having a plurality of through-holes (12) as illustrated in Fig. 7.

### (Utilization of the deodorant disk)

The monolith (11) obtained in the above example is used as a deodorant. The installation place may be the drain port of a kitchen sink or a mesh-type garbage receptacle installed in the sink.

In the location where said monolith was placed, the generation of malodors could be held to a minimum. This is because of the OYK strain inhibited growth of adventitious microorganisms responsible for putrefaction of organic matter. Thus, the monolith (11) is a porous disk comprising a conglomerate of a large number of particulate coffee grounds, thus creating an aerobic milieu, and serves as an enrichment source for OYK bacteria so that they grow vigorously. The through-holes (12) not only serve to increase the available surface area of the monolith (11) but also serve as channels for the passage of water.

The agar powder used in the above example may be partly replaced with a sizing material. The sizing material which can be used includes natural polymers such as starch and synthetic polymers such as acrylic resins. For example, 0.2 g of agar powder and 0.8 g of starch can be used in lieu of 1 g of agar powder. Moreover, coffee grounds may be partly replaced with sawdust. Sawdust serves not only to adsorb the nutrients added to water but also to increase the porosity and function as a base for growth of the bacteria.

The case of using a porous inorganic material includes the case in which a perlite matrix carrying nutrients adsorbed thereon is inoculated with the organism and the case in which a synthetic adsorbent containing a porous inorganic substance having odor-adsorbing properties, for example an inorganic particulate substance such as granular active charcoal or zeolite in a suitable proportion for enhanced adsorbency is employed. Zeolite has a large capacity to absorb trimethylamine and methylmercaptan and, in corporation with growth of the bacteria, does effectively discharge the deodorizing function. A recipe which can be used in the case of formulating zeolite is: water 100 cc, agar powder 1 g, glucose 1 g, sodium glutamate 1 g, soybean protein 1 g, coffee grounds 50 g, zeolite fine grains 5 g.

### (Prevention of growth of adventitious microorganisms in bath water)

The method of preventing growth of adventitious microorganisms in bath water which comprises cultivating the OYK strain is now described, reference being had to the accompanying drawing.

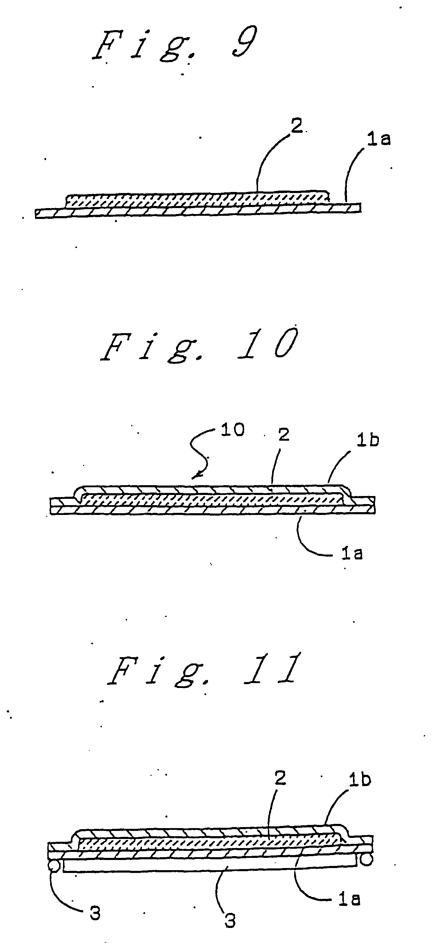

First, 1.5 g of agar powder and, as the nutrients for growth of the organism, sodium glutamate, glucose and soybean protein were dissolved in 100 cc of water and the solution was autoclaved at 120°C for 20 minutes, followed by cooling. When the mixture had gained sufficient viscosity, it was coated in a thickness of 2 mm on a rectangular nonwoven polyester fabric (1a) (15 cm × 10 cm) as shown in Fig. 9 and allowed to solidify to provide an agar medium (2). Then, on this agar medium (2), 0.1 ml of a concentrated suspension of the OYK strain (about 10⁹ cells/ml) was evenly applied.

Then, as shown in Fig. 10, a polyester nonwoven fabric (1b) was spread to cover the whole surface of the agar medium (2) and the two nonwoven fabrics were stuck together with an adhesive along the margin of the medium to provide an OYK strain-carrying support (10).

Then, by attaching a float means to the outer surface of the nonwoven polyester fabric (1a), the OYK strain-carrying support (10) can be allowed to float on the water surface. The float (3) may for example be a plastic tube having an internal diameter of 2 mm as closed at both ends, and is affixed to each of the four sides of the OYK support (10).

It is also possible to attach a cord (4) in the form of a loop to one end of the OYK-carrying support (10) and hung it on a plug (6) provided on a bathtub as illustrated in Fig. 13. In this manner, the OYK-carrying support (10) can be installed on the bathtub wall (8). As an alternative, the OYK-carrying support (10) may be stuck to the reverse side of the bathtub cover (not shown) with the aid of an adhesive tape. Moreover, in the case of a Jacuzzi, the OYK-carrying support (10) may be set in the filter at the air intake for generation of bubbles to inhibit growth of adventitious microorganisms invading from the atmosphere and, at the same time, maintain the cleanliness of the internal wall of the air supply pipe.

Aside from the above modes of use, the OYK strain can be used in a 24-hour bath (a bath such that water in the tub is not changed for every bath-taking but the dirt and filth are removed by recycling and the water temperature is kept constant to insure the comfort of taking a bath at all times) to prevent growth of adventitious microorganisms in the bath water. Thus, the OYK strain multiplies, migrates into the bath water, and further finds its way into the warm water circuit and recycling equipment. The grown OYK strain inhibits growth of adventitious bacteria (e.g. Legionella) over a long time and reduces the malodor which would be produced as the result of their growth.

The growth of adventitious bacteria such as Legionella spp. in the bathtub water can also be prevented by using the bacterial preparation obtained in the above example.

### (Application to sanitary napkins)

A suitable amount of a powder containing a large number of sporulated OYK organisms (concentration of bacteria, 1×10¹⁰ cells/g) was entrapped in the menstrual blood-absorbing member of a commercial sanitary napkin for women. This entrapping may for example be carried out by a method which comprises loading a compressed-air type duster with the bacterial powder and delivering the powder from the duster nozzle.

As the powder so entrapped comes into contact with menstrual blood, the bacteria germinate and multiply by digesting the protein contained in the menstrual blood. Since the OYK strain shows maximal activity under weakly acidic to weakly alkaline conditions at temperatures near 30°C and, moreover, the initial concentration of bacteria used is extremely high, the strain inhibits growth of other bacteria to prevent emanation of malodors and, at the same time, maintain the local region of the body clean and sanitary.

In this connection, said OYK bacterial powder can be used in admixture with a powdery enrichment source, for example soybean protein. In this case, the mixture can be directly applied to the water-absorbing member or sealed into a flat bag made of water-soluble film for insertion into the water-absorbent layer. The material for said water-soluble film includes but is not limited to starch (oblate), casein, gelatin, PVA and CMC.

The OYK bacterial powder can also be immobilized within a water-absorbent member comprising a multi-layered nonwoven fabric. Thus, the menstrual blood-absorbing member of a sanitary napkin is usually a laminate of several component nonwoven cloths and, therefore, the OYK bacteria can be deposited on one or more of the component cloth layers. In a typical procedure, a 1×10⁷ cells/ml suspension of the OYK strain is mixed with 10% of PVA (binder) and the resulting dispersion is used to print a nonwoven cloth by means of screen printing press. The print is then dried at about 60°C to cause all the OYK bacteria to sporulate. In this manner, many of the OYK spores are immobilized as intertwined with the constituent filaments of the nonwoven cloth, and this nonwoven cloth can be cut to provide component layers for the nonwoven laminate constituting said water-absorbent member.

### (Application to diapers)

In the application of the OYK strain to the baby's diaper, too, a nonwoven cloth screen-printed with an OYK bacteria-binder dispersion can be used.

The OYK strain need not necessarily be immobilized in sporulated form but can be used by the method which comprises shake-culturing the OYK strain in a 7% solution of meat extract-peptone powder (nutrient broth), immersing a nonwoven cloth in the culture fluid, and dehydrating the cloth. In this case, the OYK organisms sporulate on dehydration and get caught in position by the fibers.

### (OYK strain-incorporated film)

In an aqueous solution of starch, casein, gelatin, PVA or CMC, the sporulated OYK strain was dispersed by mixing. This dispersion was coated in a predetermined thickness on a flat surface and dried at 50∼70°C. The dried dispersion layer was peeled off from the surface to provide an OYK strain-incorporated film.

The film thus obtained can be used as a constituent layer of said menstrual blood-absorbing laminate of a sanitary napkin or used in the diaper described above.

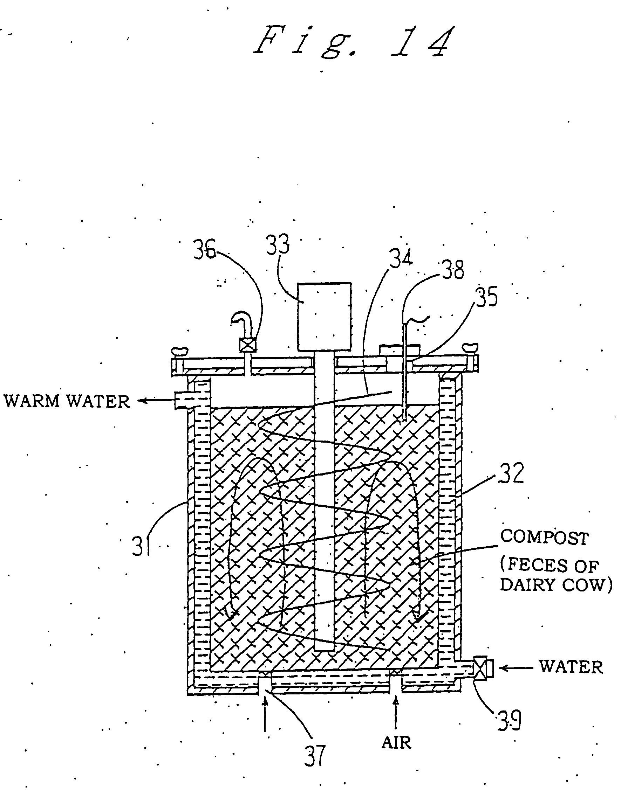

### (Utilization of metabolic energy as a thermal energy source) (Fig. 14)

In the fermentation process for compost formation from organic wastes (e.g. domestic animal manure), fermentation is accompanied by a temperature buildup. By carrying out this fermentation in a heat-insulated vessel with a removable lid (31) with moisture, nutrients (C/N ratio), aeration and temperature being controlled at the optimum levels for the most efficient fermentation, the surplus output heat of fermentation can be utilized. Usually, in the fermentation of animal excreta, the temperature increases only up to 30-50°C, but when the OYK strain or a mixed OYK-thermophilic bacterial cell preparation is added, the temperature increases rapidly in an early phase of compost formation and, as the activity of thermophiles is encouraged, rose further to 70∼100°C. The surplus heat-utilization period means the time period of compost-forming fermentation during which a temperature increase can be confirmed even if heat exchange is performed.

A temperature sensor (38) detects the temperature of the manure in the vessel and, according to the temperature data, a valve (39) is automatically operated. To prevent evaporation of water by the generation of heat, a suitable amount of water vapor can be released from an air valve (36), while nutrients and water can be added from an inlet (35) in the event of their shortage. An aeration port (37) is disposed at the floor of the heat-insulated vessel (31) in such a position or positions that there will be no considerable variation in the flow of air relative to compost volume. Moreover, the heat of fermentation in the vessel can be withdrawn as a thermal energy by a heat exchanger (32) using water, air or a liquid polymer as a medium. Furthermore, a stirring screw (34) associated with an agitation motor (33) revolves to insure uniformity of fermentation in the vessel. The frequency of stirring may be about once daily, although it depends on fermentation status.

As an alternative, the heat exchanger tubing may be installed on the floors and walls of the primary fermentation tank and secondary fermentation tank used for compost production and on the sheet covering the manure to utilize the heat of fermentation during compost formation.

In the process of fermentation of organic wastes for compost formation, the primary fermentation may be regarded as having completed when no temperature increase is detected despite positive aeration control and stirring (reshuffling). When the temperature of the load in this equipment has ceased to increase any longer, the product can be withdrawn from the vessel (31) and used directly as compost.

Generally speaking, in the fermentation for compost formation, the greater is the thermal history of a compost, the lower is its nutritive value (C/N ratio). By modulating the fermentation temperature by means of a heat-exchanger in this equipment to thereby control the process of fermentation of the load, it is possible to produce a compost which is not only fully ripe but also contains undecomposed nutrients.

Therefore, by installing a plurality of units of the above equipment, the surplus heat of fermentation from the compost-forming fermentation of organic wastes can be utilized as a source of heat required for an initial phase of fermentation or as a source of heat for the heating of the floor of an animal shed or for the melting of snow, thus contributing to a further improvement in the agricultural environment.

Moreover, if said heat is utilized for the warm water-cleaning of the floor and walls of an animal shed for dairy cow or the like, a very rewarding effect can be obtained for the prophylaxis of mastitis in the cows.

Furthermore, the equipment can be applied to an anaerobic fermentation system for extracting combustible gases from wastes. Thus, the warm water available from the above equipment can be utilized for the heating of wastes in an anaerobic fermentation tank and the efficiency of anaerobic fermentation can be improved by utilizing the bacterial preparation of the invention in a stage preceding the anaerobic fermentation, that is to say increasing the temperature of the waste to about 40°C with the aid of the bacterial preparation in advance and, then, switching the system to the anaerobic mode.

### (Utilization of the encapsulated OYK organism)

The application of the OYK strain or an OYK cell preparation containing said strain as encapsulated or sealed in a multi-layer tablet for reducing fecal odor in the human or domestic animal dosed with the capsule or tablet whether orally or as a suppository is now described.

The OYK strain by nature grows at pH 6.1 but does not grow at pH 5.4. The interior environment of the animal's stomach is strongly acidic, i.e. pH 2∼3, and when the above capsule or the like is administered orally, the bacteria contained therein are destroyed by the gastic acid. In order to have the OYK strain delivered to the intestines and reduce the fecal odor by letting the OYK strain grow in situ, it is necessary to allow the strain to reach the rectum without interfering with the function of the small intestine and utilize the feces in the rectum as organic nutrients and grow. For this purpose, the following contrivance may be utilized. Thus, the OYK strain or said OYK cell preparation is entrapped in an enteric (not soluble in the stomach but soluble in the bowels) protective film or a capsule shell, or coated with an enteric coating material, and the resulting tablets, capsules or suppositories are administered.

The above application is now described in further detail.

For example, 14 weight parts of gelatin, 3 weight parts of glycerin, 3 weight parts of low-methoxylated pectin and 80 weight parts of purified water are uniformly blended to prepare a liquid covering composition. This covering composition is extruded from an annular orifice and concurrently a kneaded composition of the OYK strain or OYK cell preparation is extruded from an inner orifice disposed in concentric relation to said annular orifice and the resulting composite jet extrudate is chilled in a cooling bath to provide an encapsulated product having a grain size of 0.5∼10 mm in diameter. This product is immersed in an aqueous solution of calcium chloride, dried, and rinsed to provide enteric soft capsules.

## Claims

1. A novel nonhemolytic Bacillus subtilis-related microorganism characterized in that it has a cell volume 4-fold as large as that of the type culture strain of Bacillus subtilis (IFO 3134) and, when shake-cultured in nutrient broth at 37°C, is capable of proliferating from an initial population of 10³ cells/ml to a population of not less than 10⁷ cells/ml in 4 hours owing to substantial absence of an induction period following initiation of culture, with the metabolic energy produced in growth of said microorganism reaching not less than 10,000-fold as high as that of said type culture strain of Bacillus subtilis as a synergistic effect.

2. A novel microorganism characterized by its being a nonhemolytic Bacillus subtilis-related microorganism of the genus Bacillus which has antimicrobial activity against at least Staphylococcus aureus, Klebsiella pneumoniae, meticillin-resistance S. aureus, pathogenic E. coli O-157, Legionella spp., Pseudomonas aeruginosa, Fusarium spp., koji mold (Aspergillus oryzae), blue mold (Penicillium spp.), Trichophyton spp., and Rhyzopus spp.

3. The novel microorganism according to Claim 1 or 2 which is at least one member selected from the group consisting of Bacillus sp. OYK-01-600 (FERM BP-6394), Bacillus. sp. OYK-03-600 (FERM BP-6395) and Bacillus sp. OYK-04-000 (FERM BP-6396).

4. The novel microorganism according to any of Claims 1∼3 which is for the production of a compost.

5. A compost as produced with the aid of the novel microorganism defined in Claim 4.

6. A bacterial cell preparation characterized by its comprising the novel microorganism as entrapped in, deposited on, or incorporated in an enrichment source.

7. The bacterial cell preparation according to Claim 6 wherein said enrichment source is predominantly composed of protein.

8. The bacterial cell preparation according to Claim 7 wherein at least a portion of said enrichment source is comprised of a porous substance.

9. The bacterial cell preparation according to Claim 8 wherein said porous substance is at least one member selected from the group consisting of coffee grounds, edible oil extraction residues, food extraction residues such as soybean curd refuse, beer cake, coconut cake, fruit cake, etc., and plant fragments such as sawdust and hull chaff.

10. The bacterial cell preparation according to any of Claims 6∼9 further containing thermophilic bacteria growing in a high temperature region not less than 50°C, such as actinomyces and hyphomyces.

11. The bacterial cell preparation according to any of Claims 6∼10 which is for the production of a compost.

12. A compost as produced with the aid of the bacterial cell preparation defined in Claim 11.

13. A warming method which comprises using the bacterial cell preparation defined in any of Claims 6∼10 to exploit the metabolic energy produced in growth of the novel microorganism contained therein as a thermal energy source.

14. A heating equipment using the bacterial cell preparation defined in any of Claims 6∼10 to exploit the metabolic energy produced in growth of the novel microorganism contained therein as a thermal energy source.

15. A pharmaceutical preparation comprising the novel microorganism defined in any of Claims 1∼3 as entrapped in an enteric protective film or an enteric capsule shell.

16. A warming device comprising the bacterial cell preparation defined in any of Claims 6∼10.

17. An antimicrobial article comprising the bacterial cell preparation defined in any of Claims 6∼10.
